# EUROPEAN PATENT SPECIFICATION

(11) **EP 2 619 582 B1**
(45) Date of publication and mention of the grant of the patent: **07.01.2015**
(21) Application number: 11758243.7
(22) Date of filing: 22.09.2011
(51) Int. Cl.: G01N 33/566, G01N 33/574, G01N 33/68, G01N 33/74, C12Q 1/68

(54) **BREAST CANCER DIAGNOSTICS**
BRUSTKREBSDIAGNOSE
DIAGNOSTIC DU CANCER DU SEIN

(30) Priority: 22.09.2010 EP 10178345
(43) Date of publication of application: 31.07.2013
(73) Proprietor: IMBA-Institut für Molekulare Biotechnologie GmbH, 1030 Wien (AT)
(72) Inventor: PENNINGER, Josef, A-1130 Vienna (AT); SCHRAMEK, Daniel, A-1190 Vienna (AT); SCHETT, Georg, A-4400 Steyr (AT); WIDSCHWENDTER, Martin, Tonbridge Kent TN10 3A (GB); JACOBS, Ian J., Eynsford Kent DA40HT (GB); MENON, Usha, London Greater London N21 1SW (GB)
(74) Representative: Sonn & Partner Patentanwälte
(86) International application number: PCT/EP2011/066514
(87) International publication number: WO 2012/038505

(56) References cited:
- WO-A1-00/43553
- WO-A2-2005/060627
- TERPOS EVANGELOS ET AL: "Soluble receptor activator of nuclear factor kappaB ligand-osteoprotegerin ratio predicts survival in multiple myeloma: proposal for a novel prognostic index.", BLOOD, vol. 102, no. 3, 1 August 2003 (2003-08-01), pages 1064-1069, XP002621518, ISSN: 0006-4971 cited in the application
- DATABASE MEDLINE [Online] US NATIONAL LIBRARY OF MEDICINE (NLM), BETHESDA, MD, US; July 2004 (2004-07), HASHIMOTO TOSHIHIRO ET AL: "[Biochemical markers in bone metastasis].", XP002621519, Database accession no. NLM15272580 cited in the application & GAN TO KAGAKU RYOHO. CANCER & CHEMOTHERAPY, vol. 31, no. 7, July 2004 (2004-07), pages 1027-1033, ISSN: 0385-0684 cited in the application
- BELEUT MANFRED ET AL: "Two distinct mechanisms underlie progesterone-induced proliferation in the mammary gland.", PROCEEDINGS OF THE NATIONAL ACADEMY OF SCIENCES OF THE UNITED STATES OF AMERICA, vol. 107, no. 7, 16 February 2010 (2010-02-16), pages 2989-2994, XP002621520, ISSN: 1091-6490 cited in the application
- LEIBBRANDT A ET AL: "ESCI award lecture: from a little mouse to rationale medicine for bone loss", EUROPEAN JOURNAL OF CLINICAL INVESTIGATION, vol. 39, no. 10, 1 October 2009 (2009-10-01), pages 842-850, XP009144002, WILEY-BLACKWELL PUBLISHING LTD, GB ISSN: 0014-2972, DOI: 10.1111/J.1365-2362.2009.02215.X [retrieved on 2009-09-15] cited in the application
- REID PENNY E ET AL: "Breast cancer cells stimulate osteoprotegerin (OPG) production by endothelial cells through direct cell contact.", MOLECULAR CANCER, vol. 8, no. 49, 2009, pages 1-10, XP002621521, ISSN: 1476-4598 cited in the application
- BO-YING BAO ET AL: "Clinical Significance of Tumor Necrosis Factor Receptor Superfamily Member 11b Polymorphism in Prostate Cancer", ANNALS OF SURGICAL ONCOLOGY, vol. 17, no. 6, 4 March 2010 (2010-03-04), pages 1675-1681, XP019811562, SPRINGER-VERLAG, NE ISSN: 1534-4681 cited in the application
- SCHRAMEK DANIEL ET AL: "Osteoclast differentiation factor RANKL controls development of progestin-driven mammary cancer.", NATURE, vol. 468, no. 7320, 4 November 2010 (2010-11-04), pages 98-102-METHODS, XP002621522, ISSN: 1476-4687
- PARK HYE-RIM ET AL: "Expression of osteoprotegerin and RANK ligand in breast cancer bone metastasis", JOURNAL OF KOREAN MEDICAL SCIENCE, KOREAN ACADEMY OF MEDICAL SCIENCE, SEOUL, KR, vol. 18, no. 4, 1 August 2003 (2003-08-01) , pages 541-546, XP009143976, ISSN: 1011-8934
- Gaoping Chen ET AL: "Expression of RANKL/RANK/OPG in primary and metastatic human prostate cancer as markers of disease stage and functional regulation", Cancer, vol. 107, no. 2, 15 July 2006 (2006-07-15) , pages 289-298, XP055047453, ISSN: 0008-543X, DOI: 10.1002/cncr.21978
- Ki Won Oh ET AL: "Circulating osteoprotegerin and receptor activator of NF-kappaB ligand system are associated with bone metabolism in middle-aged males", Clinical Endocrinology, vol. 62, no. 1, 1 January 2005 (2005-01-01), pages 92-98, XP055116143, ISSN: 0300-0664, DOI: 10.1111/j.1365-2265.2004.02179.x
- PIETRAPERTOSA A C ET AL: "Osteoprotegerin and RANKL in the pathogenesis of osteoporosis in patients with thalassaemia major", PANMINERVA MEDICA, EDIZONI MINERVA MEDICA, TORINO, IT, vol. 51, no. 1, 1 March 2009 (2009-03-01), pages 17-23, XP009177518, ISSN: 0031-0808
- Rina Ohwada ET AL: "The Relationship between Serum Levels of Estradiol and Osteoprotegerin in Patients with Anorexia Nervosa", Endocrine Journal, vol. 54, no. 6, 1 January 2007 (2007-01-01), pages 953-959, XP055116147, ISSN: 0918-8959, DOI: 10.1507/endocrj.K07-034

## Description

The present invention relates to the field of breast cancer diagnostics.

Breast cancer is one of the most common cancers in humans and will on average affect up to one in eight women in their life time in the US and Europe. The Women's Health Initiative (WHI) and the Million Women Study have shown that hormone replacement therapy (HRT) is associated with an increased risk of incident and fatal breast cancer. In particular synthetic progesterone derivatives (progestins) such as medroxyprogesterone acetate (MPA), used in millions of women for HRT and contraceptives, markedly increase the risk of developing breast cancer.

Receptor Activator of NF-κB Ligand (RANKL, also known as ODF, TRANCE, OPGL, TNFSF11) and its receptor RANK (TRANCE-R, TNFRSF11A) are essential for the development and activation of osteoclasts. RANKL inhibition is at the verge of approval for potentially millions of patients to prevent bone loss. RANK and RANKL have been cloned and characterized (US 6,017,729, EP 0 873 998, EP 0 911 342, US 5,843,678, WO 98/46751, WO 98/54201). Both RANKL and RANK expression have been observed in primary breast cancers in humans and breast cancer cells lines and it has been proposed that the RANKL/RANK system can regulate bone metastases of epithelial tumors¹⁴ without an effect on proliferation or death susceptibility.

Terpos et al., Blood 102 (3) (2003): 1064-1069, describes markers for the prognosis of the development of multiple myeloma, a cancer causing bone lesions and interfering with the production of blood cells in the bone marrow.

Hashimoto et al., Cancer & Chemotherapy 7 (31) (2004): 1027-1033 (abstract), mentions that bone metabolic markers including soluble RANKL and OPG, reflecting osteoclasts and osteoblast activity, provide information on the severity of bone diseases and prognosis.

WO 2005/060627 A2 relates to methods for determining the risk of non-traumatic bone fracture by measuring the level of RANKL. Tools for use in such a method are e.g. antibodies against RANK or RANKL

WO 00/43553 A1 discloses screening methods of women for breast cancer by determining a level of an estrogen-related marker. The markers determined in this method are an aromatase enzyme, aromatase activity, a byproduct of estrogen synthesis and a protein effector acting upstream of estrogen synthesis, obtained from breast ductal fluids.

Beleut et al., Proceedings of the National Academy of Sciences of the United States of America 107 (7) (2010): 2989-2994, provides an investigation of mechanisms leading to mammary gland proliferation. Predominantly, progesterone drives proliferation of mammary epithelial cells.

Leibbrandt et al., European Journal of Clinical Investigation 39 (10) (2009): 842-850, is a review article on the functions of RANK, RANKL and OPG on osteoblasts and osteoclasts.

Reid et al., Molecular Cancer 8 (49) (2009): 1-10, discloses stimulation of OPG production by endothelial cells.

Bo-Ying et al., Annals of Surgical Oncology 17 (6) (2010): 1675-1681, describes the identification of a certain polymorphism in the TNFRSF11B gene that is involved in increased bone metastasis of prostate cancer.

Park et al. (2003) contains a discussion of the role of OPG and RANKL in breast cancer bone metastasis.

Chen et al. (2006) disclose that RANKL/RANK/OPG expression is increased in aggressive metastatic prostate carcinoma.

It is a goal of the present application to provide diagnostic methods and means for identifying cancer.

The present invention relates to the specific role of RANKL in cancer, its use to diagnose and predict cancer and cancer development.

According to a first aspect, the present invention provides a method of detecting a cancer or predicting a patient developing cancer, wherein said cancer is breast cancer, comprising determining RANKL activity and the amount of OPG in a sample of said patient. The present invention is further defined in the claims.

According to the present invention it was discovered that the RANK/RANKL pathway is associated with cancer development and can be used to detect cancer and predict the onset of cancer. It was found that RANKL is responsible for protecting cells from cancerogenous mutations as it prevents cell death after such mutations induced by DNA damage. Survival of cells despite transforming mutations is one key property of cancer cells. The newly discovered role of RANKL in this activity allows the correlating RANKL activity and/or OPG concentration with cancer development. Thus, the likelihood of a patient having or developing a cancer can be estimated. The present invention also relates to the method of identifying or predicting the risk of developing cancer cells in a subject by determining RANKL activity or OPG concentration in a sample of the subject.

According to the present invention "predicting" shall not be construed in an absolute sense, i.e. in the meaning, that with 100% certainty it can be predicted that a patient will definitely develop cancer but the invention relates to estimating the risk of a patient for developing cancer. Similar, "detecting a breast cancer" also does not claim that all cancer patients can be identified by determining the RANKL activity but the patient may have a high change of having a cancer. Therefore, determining the RANKL activity can be a first lead to identify a breast cancer, possibly followed by more invasive tests.

The invention is based on the result that RANKL activity - as well as the concentration of its natural ligand OPG - can be correlated with cancer occurrence and development and therefore, in a preferred embodiment such a correlation may be included according to the inventive method. It is e.g. possible to correlate data of negative control (e.g. of patients who do or did not develop cancer) and/or positive control (of patients who did develop cancer) data and compare such negative or positive control data with the RANKL activity of the sample of the patient. Furthermore, in order to improve diagnostic power it is possible to monitor the RANKL activity, i.e. measure the RANKL activity in samples of the patient of different time points, e.g in certain intervals of at least or at most e.g. 1 week, 2 weeks, 3 weeks, 4 weeks, 5 weeks, 6 weeks, 7 weeks, 8 weeks, 1 month, 2 months, 3 months, 4 months, 5 months, 6 months, 7 months, 8 months, 9 months, 10 months, 11 months or 12 months or more. Changes in the RANKL activity at two or more timepoints can indicate cancer development. In particular embodiments it is preferred to monitor RANKL activity values at the same time in the menstrual circle as RANKL activity can be influenced by female sexual hormones. For example, it is preferred that RANKL activity of the sample is measured at a fixed amount of days after ovulation.

In preferred embodiments the breast cancer is a breast cancer dependent on hormones for growth. Such hormones may be sexual hormones, such as female sexual hormones like progesterone or an estrogene. The cancer cells may have hormone receptors, especially progesterone receptors or estrogene receptors. Examples of estrogene receptors are ESR1 (comprising ER alpha chains), ESR2 (comprising ER-beta chains) or heteromeric receptos, such as of mixed ER-alpha and ER-beta chains. Presence of such receptors may indicate a requirement of hormone signalling in the cell. In particular, this signalling may be RANKL-mediated. Activation of RANKL by hormones protects the cancer or a pre-cancerous cell from DNA damage induced cell death. Thus these hormones may support cancer via increased RANKL activity that can therefore be used to diagnose or predict cancer according to the present invention.

According to the invention the cancer may be a primary cancer. As shown herein even up to two years ahead of clinical cancer manifestation, a cancerous development can be diagnosed and thus the cancer can be predicted by determining RANKL activity. Therefore the primary cancer can be diagnosed or predicted. In addition it is also possible to diagnose or predict the development of re-occuring cancer.

According to the invention it was found that RANKL levels distinctively differ from negative controls up to two years ahead of a developing cancer. For example it was found that RANKL levels in serum are significantly increased 12 to 24 months before the onset of clinical manifest cancer, that can e.g. be diagnosed by a conventional biopsy. Surprisingly, RANKL serum levels are decreased 5 to 12 months ahead of a manifest cancer. By comparison with positive control data of samples taken ahead of patients who at a certain time did develop cancer it is possible to correlate the data of the sample and predict future cancer development and even the time until clinical symptoms of a cancer can be detected by conventional means, such as by biopsy.

RANKL is a known ligand of cell surface receptor RANK that regulates function of dendritic cells and osteclasts. According to the present invention, a further mechanism in the development of cancer has been discovered. RANKL drives hormone influenced cancer development. Such hormones may be of the normal hormonal background in any individual or may have been artificially administered (such as in hormone replacement therapies, in menopause treatment or as contraceptive). Furthermore, the cellular mechanism and activity of this ligand in cancer has been investigated and characterized. The effect of RANKL, "RANKL activity", includes binding of RANKL to RANK and its resulting activation. RANK in term further activates IKKα, IκBα, P-NFκB and cyclinD1 or activates the Id2-p21, MAPK Erk and p38 pathway. Any upregulation in this pathway due to RANKL can be used as indicator for the inventive diagnostic method. Likewise modifying activity of any of these factors can be used for a therapeutic method that is further described herein. Therefore, any upregulation or activation of any member of this pathway can use to estimate the "RANKL activity". Most of these proteins are intracellular and it is possible to evaluate their concentration by using cellular samples. Alternatively or in addition to estimating the protein concentration it is possible to determine expression of these proteins, e.g. by determining mRNA concentrations in the cells, in particular breast tissue cells. Therefore the present invention comprises determining the RANKL activity by determining signalling of any one of RANK, IKK-alpha, IkB-alpha, P-NF-kappa-B, CyclinD1 (Id2-p21, MAPK Erk or p38). Signalling and activation of any of these factors can e.g. be measured by detecting and/or quantifying the amount of activated RANK, IKK-alpha, IkB-alpha, P-NF-kappa-B and/or CyclinD1 or Id2-p21, MAPK Erk and/or p38. Activated forms of these proteins can e.g. be determined by determining phosphorylated versions of these proteins or by determining protein aggregates. As always a comparison to a negative or positive control may help in identifying increased activation of one or more of these proteins. For accessibility reasons, however, it is preferred to determine the RANKL concentration in blood or serum samples of a patient.

RANK is not the only receptor for RANKL. Osteoprotegin (OPG) is a secreted decoy receptor that can reduce the RANKL activity (binding of RANKL to RANK and its signaling pathway via IKKα, IκBα, P-NFκB and cyclinD1). In addition, RANKL upregulates OPG expression and may lead to increased OPG concentration, in particular in a blood or serum sample. OPG may reduce the concentration of free RANKL by binding to it. In some embodiments of the present invention, the RANKL concentration may be determined in its free, soluble form. In other embodiments, the total RANKL concentration (free RANKL in addition to OPG bound RANKL) can be an indicator of cancer. Although, the activity of RANKL is a cause of breast cancer development, upregulation of RANKL concentrations which in turn may also upregulate its regulatory ligand OPG that can act as diagnostic marker to diagnose or predict cancer. Therefore, for diagnostic purposes, the term "determining RANKL activity" includes both the determination of any upregulation in the RANKL signalling pathway or in any RANKL concentration. In particular preferred embodiments a combination of OPG and RANKL is used as marker for the inventive predictions or diagnosis, especially the ratio in a sample between RANKL as OPG amounts may be used. Increased OPG concentrations that result in decreased free soluble RANKL most likely is a result of a previous phase of increased RANKL concentrations (without an increased OPG buffering effect) that might already have initiated the first steps in cancer development. Therefore, in line with the findings of different RANKL concentrations during different stages of cancer onset, leading to a development of a clinical cancer, OPG is a similar inverse marker of cancer development. Therefore the present invention relates to the use of OPG as a diagnostic marker for the diagnosis or prognosis of cancer.

In preferred embodiments an increased RANKL activity or RANKL serum concentration is used to estimate that a cancer will develop (most likely) within 12 to 24 months. A decreased serum RANKL concentration can be used to predict the onset of cancer within 5 to 12 months, both increase and decrease are preferably detected in comparison to a negative control. Preferably a decrease of RANKL and in an increase of OPG is used as indication for the inventive diagnosis or predictions/prognosis. In preferred embodiments, comparative positive controls are used to estimate the time until a cancer will most likely develop. Negative and positive control values can be used in a correlation analysis, preferably follow by statistical analysis to determine statistical significance, e.g. comparing groups using Student's T-test.

The RANKL activity is preferably determined by determining the amount of RANKL and/or RANK in the sample. Although, any signalling factor within the RANKL/RANK cascade can be analysed to predict the onset of breast cancer development, it is preferred to determine the amount of RANKL and/or RANK itself. Usually the amount of RANKL or RANK refers to a concentration within the sample volume or alternatively as an amount per cell in the sample. In particular in the case of RANKL it is preferred to determine solute RANKL concentrations, particular in a blood or serum sample.

Cellular samples preferably comprise cells of the patient of the tissue that may be diagnosed or indicated for further cancer development. Preferably the cells comprise breast cells. The amount of concentration of RANKL, RANK or any further member of the RANKL signalling pathway can be estimated by known methods in the art, in particular by binding a ligand and detecting such binding events. Preferred ligands are antibodies directed against RANKL or RANK or at any cellular protein of the signalling pathway. Anti-RANKL-antibodies are commercially available, e.g. Denosumab, and are disclosed in the US 2008/107597. In order to determine intracellular proteins it is usually necessary to break the cell-membrane or homogenize a cell sample or using intracellular labels. Preferably binding events are detected by using a label, especially fluorescence labels.

In further embodiments it is possible to estimate the RANKL activity by determining the concentration of RANKL mRNA levels or the mRNA levels of any factor in the RANKL signalling pathway, including RANK, IKKα, IκBα, P-NFκB and cyclinD1, alone or in combination of one or more of these factors, preferably of RANKL. Increased expression of any of these factors and in particular of RANKL is a direct indicator of increased RANKL activity that is the cause of developing cancer and therefore can be used to diagnose or predict cancer.

Furthermore, the RANKL activity can be correlated with the progression and expansion of cancer, especially a hormone-driven cancer like breast cancer. The cancer may be of epithelial origin. Preferably such correlations are made in comparison with positive control samples of patients who developed cancer which progressed at a specific rate.

The patient is preferably a mammal, in particular preferred a primate, even more preferred a human, in particular a female. The patient might have or might have had a therapy with female sexual hormones.

Progesterone and in particular its synthetic derivatives (progestins) are used in combined hormone replacement therapies (HRT) in postmenopausal women, initially to decrease the risk of developing estrogen-driven endometrial cancer and to ameliorate menopausal symptoms. Medroxyprogesterone acetate (MPA) is the most frequently and longest used progestin in hormonal contraceptives and is commonly used for HRT.

It was found according to the present invention that RANKL activity in cancer development is influenced by sexual hormones, including female sexual hormones like estrogen or progesterone, inparticular by progesteron or its dervatives (progestins). Therefore, in preferred embodiments the patient is treated by a hormone, preferably receives hormone replacement therapy, preferably with progesterone or a progestin, or with a hormone contraceptive. Examples of progestins are medroxyprogesterone (or its acetate, e.g. the 17-acetate), norethisterone, ethisterone, norethynodrel, norethindrone acetate, ethynodiol diacetate, levonorgestrel, norgestrel, desogestrel, gestodene, norgestimate, drospirenone, dienogest, nesterone, nomegestrol acetate, trimegestrone, tanaproget, megestrol acetate, pranone, etonogestrel. The hormone or derivative preferably has progestinic effects.

Natural hormone levels can be sufficient to induce a cancerous RANKL protection of cancerous mutations in cells. Hormones may also be artificially increased in a subject. In hormone replacement therapies or by using hormone contraceptives, the hormone level, in general of sexual hormones, is upregulated leading to increased progesterone levels that could be tied to development of cancer via the RANKL pathway according to the present invention. Therefore, patients which receive or have been treated by a hormone or hormone contraceptive are at an increased risk of developing cancer. For these patients, determining the RANKL activity as described above is particularly predictive of cancer, cancer development or cancer progression.

In particular, it was found that by including determining a hormone of the menstrual cycle or female sexual hormone or function derivative thereof (such as progestins) and preferably including this data in a correlation together with the value of the RANKL activity, in particular in comparison with a negative and/or positive controls, the predictive value of the inventive diagnosis, prediction of cancer or a prediction of progression of cancer can be improved. In addition, or as an alternative, it is also possible to determine the concentration of a RANKL ligand in the sample. This allows a correction of the determination of the free serum RANKL concentration by including information of RANKL possibly bound to a ligand. A preferred ligand of RANKL is a serum ligand of RANKL, such as OPG. Therefore, in further preferred embodiments the inventive methods comprise determination of the serum concentration of a hormone of the menstrual cycle or a functional derivative thereof or a RANKL ligand in a sample of the patient. The RANKL ligand may be a RANKL regulatory protein that is regulated by the concentration of RANKL in vivo, such as OPG.

An example of such a hormone is progesterone and examples of progesterone derivatives are progestins, such as medroxyprogesterone acetate (MPA). In particular high progestin or progesterone levels (e.g. serum concentration >0.3ng/ml) and RANKL activity can be correlated with cancer development. Therefore in preferred embodiments the present invention provides determining the risk of developing breast cancer by estimating both the values of RANKL activity and progesterone (and/or progestin levels), especially serum concentrations thereof.

Alternatively, or in addition thereto, the present invention also includes determination of the amount of osteoprotegerin (OPG) in a sample of said patient, preferably in a serum sample. Osteoprotegerin is a secreted natural ligand of RANKL that can reduce the free serum RANKL concentration. In addition OPG may be upregulated in response to increased RANKL concentrations in the serum and therefore OPG is an alternative or additional indicator of cancer, or of the risk of developing cancer.

In particular it is preferred to determine both the RANKL activity and the concentration of OPG and correlate both concentrations and activities in comparison with negative and/or positive control samples for the inventive diagnosis or prediction of cancer. For such a correlation any addition, substraction or ratio between osteoprotegerin and RANKL can be used. In preferred embodiments (new-onset) cancer is diagnosed or predicted by determining the ratio of osteoprotegerin to RANKL. Surprisingly such a ratio achieved very high statistical significance in serum data by using serum concentrations of osteoprotegerin and serum concentrations of free soluble RANKL. Free soluble RANKL is a preferred species of RANKL determined according to the present invention.

Means to detect RANKL include binding ligands, especially anti-RANKL antibodies, that are specific for RANKL. Suitable antibodies are disclosed in US 2008/107597 and are commercially available, e.g antibody Denosumab. "Anti-RANKL-antibody" includes any functional equivalents and derivatives thereof, including antibody fragments such as Fab, F(ab)2, Fv, or single chain antibodies (scAb). Antibodies specifically binding the RANKL activity associated proteins and factors, especially RANKL and RANK and any proteins in the RANKL signalling pathway, are also encompassed by the invention. The antibodies may be produced by immunization with full-length protein, soluble forms of the protein, or a fragment thereof. The antibodies of the invention may be polyclonal or monoclonal, or may be recombinant antibodies, such as chimeric antibodies wherein the murine constant regions on light and heavy chains are replaced by human sequences, or CDR-grafted antibodies wherein only the complementary determining regions are of murine origin. Antibodies of the invention may also be human antibodies prepared, for example, by immunization of transgenic animals capable of producing human antibodies (WO 93/12227). The antibodies are useful for detecting RANKL in biological samples, thereby allowing the identification of cells or tissues which produce the protein.

The present invention also provides kits comprising a detection agent for RANKL, a detection agent for OPG, and a detection agent for progesterone or a progestin.

Kits may comprise additives, detecting reagents, wash solutions and/or buffers (Tris, acetate or phosphate buffers) having various pH values and ionic strengths, solubilizer such as Tween or Polysorbate, carriers such as human serum albumin or gelatin, preservatives such as thimerosal or benzyl alcohol, and antioxidants such as ascorbic acid or sodium metabisulfite. Selection of a particular kit composition will depend upon a number of factors, including the sample being used.

A "detection agent" relates to means for detection and/or quantifying a certain analyte in a sample.

The physiological RANKL ligand is OPG. The hormone may be a progestin, especially progesterone.

The RANKL detection agent can be a RANKL binding ligand, including synthetic ligands and small molecules. Preferably the RANKL binding ligand is an anti-RANKL-antibody. The RANKL binding ligand can be labelled, such as by fluorescence or a radioactive isotope.

This kit may be used for the inventive method of determining, predicting or prognosing cancer, cancer development or of identifying cancer cells as described in detail above.

The present invention is further illustrated by the following figures and examples, without being limited to such specific examples.

### Figures:

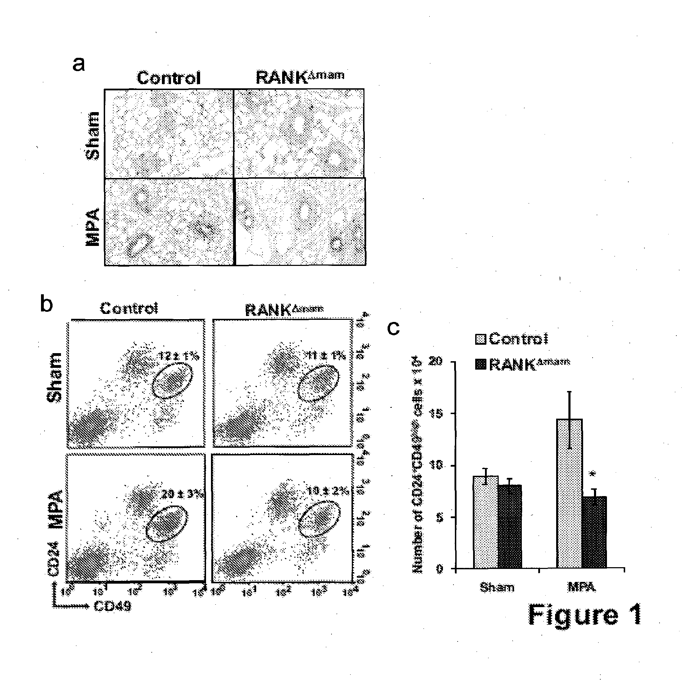

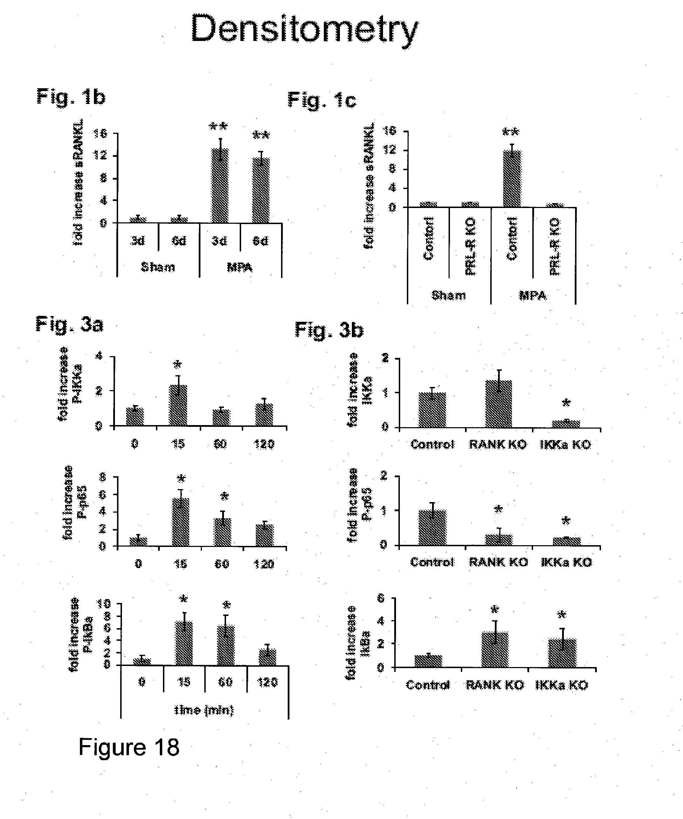

Figure 1. The progesterone-derivative MPA triggers *in vivo* RANKL expression and mammary epithelial cell proliferation via RANK.
   **a,** Epithelial proliferation in mammary glands of control littermates and RANK^{Δmam} females 3 days after sham treatment and MPA implantation. Proliferation was determined by *in situ* Ki67 immunostaining**. b,c,** Marked increase of the stem cell-enriched CD24⁺CD49^{high} population (MaSC) in MPA-treated mammary glands in control but not in RANK^{Δmam} mammary glands**. b,** Representative FACS profiles showing CD24 and CD49 expression of lineage negative (CD31- (endothelial cells) CD45⁻ (hematopoietic cells) TER199-(erythroid cells)) of mammary MaSCs from MPA- or sham-treated 8-week old virgin females**. c,** Quantification of MaSC-enriched CD24⁺CD49^{high} population from mammary glands of MPA- or sham-treated virgin RANK^{Δmam} and littermate control females. For all MaSCs experiments mice were treated with MPA for 3 days. n=4 per group +/- sem. * P < 0.05; *** P < 0.001 (Student's t-test).

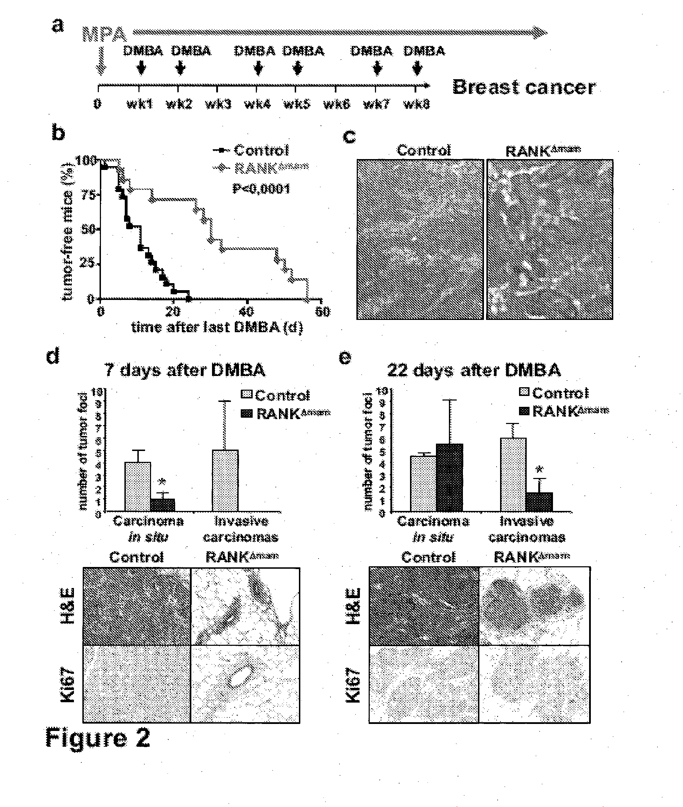

Figure 2. RANK controls the incidence and onset of progestin-driven mammary cancer.
   **a,** MPA/DMBA carcinogenesis scheme. Nulliparous, six-week old female mice were s.c. implanted with MPA pellets and treated orally with DMBA as indicated for 8 week**. b,** Onset of palpable mammary tumors in MMTV-Cre rank^{floxed/Δ} females (RANK^{Δmam}) (n=14) and age-matched littermate control females (n=19) treated with MPA pellets and DMBA as indicated in Fig. 2a. Data are shown as percentage of tumor free mice after the last DMBA challenge. Median tumor onset for controls was 11 days after last DMBA treatment and 30 days for RANK^{Δmam} females**. c,** Representative histological sections of mammary tumors isolated from control littermate and RANK^{Δmam} females 22 days after the last DMBA treatment. Cytokeratin5 staining is shown. Magnifications X 20 d,e, Numbers of carcinomas *in situ* and invasive mammary cancers in control and RANK^{Δmam} females on day 7 **(d)** and day 22 **(e)** after the final DMBA treatment. Data are shown as mean values per mouse +/- sem. n=3 mice per genotype. All 10 mammary glands were analysed for each mouse. * P < 0.05 (Student's t-test). Bottom panels show representative histological sections with typical invasive adenocarcinomas in the control females. For RANK^{Δmam} females, normal acinar morphology (day 7) and a carcinoma in situ (day 22) are shown. H&E stained sections and immunostaining for the proliferation marker Ki67 are shown. Magnifications X20.

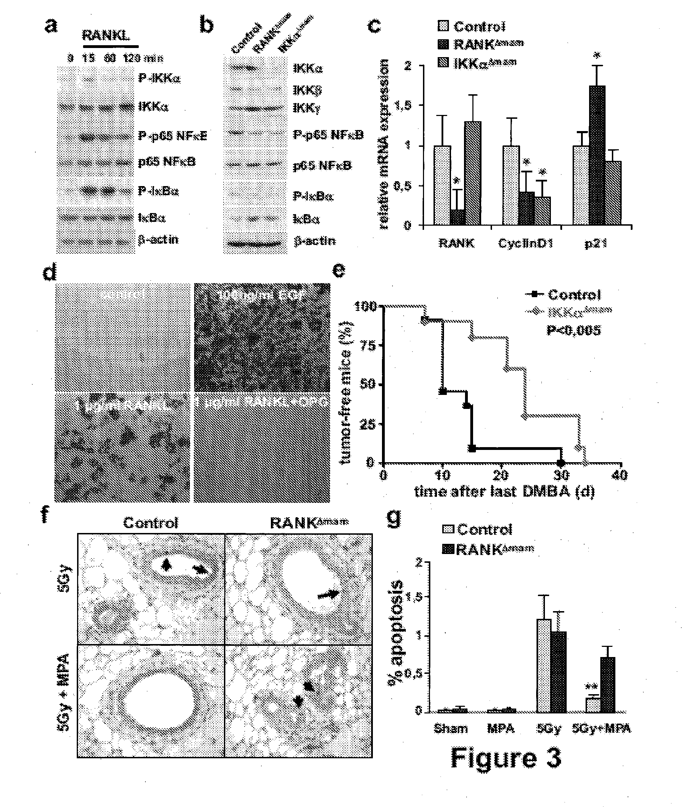

Figure 3. RANK induces NFκB signaling, anchorage-independent growth, and protects from radiation-induced epithelial apoptosis.
   **a,** Western blotting for phosphorylated (P) IKKα, total IKKα, phosphorylated (P) p65 NFκB, total p65 NFκB, phosphorylated (P) IκBα, and total IκBα in isolated primary mouse mammary gland epithelial cells (MECs) in response to RANKL stimulation (1µg/ml). β-actin is shown as loading control**. b**, Western blotting for IKKα, IKKβ, IKKγ, phosphorylated (P) p65 NFκB, total p65 NFκB, phosphorylated (P) IκBα, and total IκBα in pooled late stage mammary adenocarconimas (n=4 for each lane) that developed in control, RANK^{Δmam}, and IKKα^{Δmam} females. β-actin is shown as loading control **c,** Expression of RANK, CyclinD1, and p21 mRNA in late stage mammary adenocacinomas that developed in control, RANK^{Δmam}, and IKKα^{Δmam} females. Expression was determined by qRT-PCR. Data are mean values +/- sem. n=4 per group**. d,** Soft-Agar Colony Formation Assay. Growth of human SKBR3 breast cancer cells in soft agar in response to stimulation with RANKL (1µg/ml) or EGF (100ng/ml). Anchorage-independent, macroscopic colonies formed after 18 days in culture with RANKL, which was prevented by the decoy receptor OPG (1µg/ml). Controls were unstimulated SKBR3 cells**. e,** Onset of palpable mammary tumors in IKKα^{Δmam} (n=10) and age matched littermate control (n=11) females treated with MPA pellets and DMBA. Data are shown as percentage of tumor free mice after the last DMBA challenge. Median tumor onset for controls was 10 days after last DMBA treatment and 24 days for IKKα^{Δmam} females**. f,g** γ-irradiation (5 Gray) induced mammary epithelial cell apoptosis in control and RANK^{Δmam} female littermates either sham operated or implanted with a MPA pellet. Apoptosis was detected by immunostaining for active Caspase 3. **f,** Apoptotic nuclei of epithelial cells (arrows) are shown for representative mammary gland sections. Magnifications X 40. **g,** Quantification of mammary epithelial apoptosis. A minimum of 5000 nuclei was counted for each mouse. Results shown are mean values +/- sem. n = 3 mice per group. * P < 0.05; ** P< 0.02 (Student's t-test).

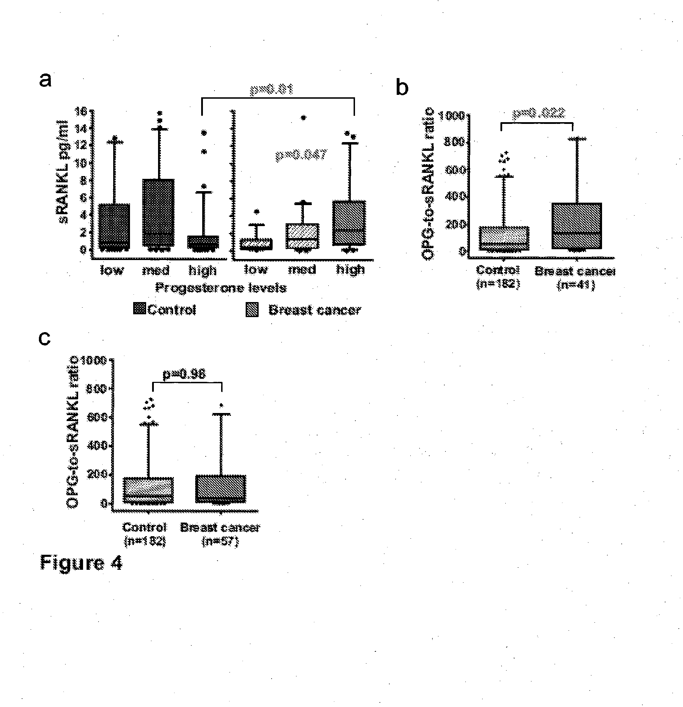

Figure 4. RANK controls cancer stem cell self renewal and RANKL/OPG serum levels in human breast cancer patients.
   **a,** Analysis of sRANKL and progesterone in serum samples from the same cohorts as in e. Whereas no association between serum progesterone and RANKL could be found in the control group (p = 0.43) there is a clear association between RANKL and serum progesterone levels in women who developed breast cancer 12-24 months after serum sampling. p = 0.047 (Spearman rank test). There was no correlations of serum progesterone and RANKL levels in women who developed breast cancer within 6-12 months after the serum was tested (p = 0.76). Data are shown as a function of serum progesterone (low <0.2 ng/ml; medium 0.2-0.3ng/mL; high >0.3 ng/mL)**. b,** Analysis of OPG-to-sRANKL ratios in prospectively collected UKCTOCS serum samples from 182 healthy postmenopausal women who did not develop breast cancer during their follow up and 41 healthy age-matched women who developed ER positive breast cancer 5-12 months after their serum was collected. Box plots show significantly higher OPG-to-sRANKL ratios in women who developed breast cancer within the first year after serum sampling. p = 0.022 (Mann Whitney U test)**. c,** Analysis of OPG-to-sRANKL ratios in UKCTOCS serum samples from 182 healthy postmenopausal women and 57 healthy age-matched women who did develop ER positive breast cancer 12-24 months after their serum was collected. No significant difference was observed.

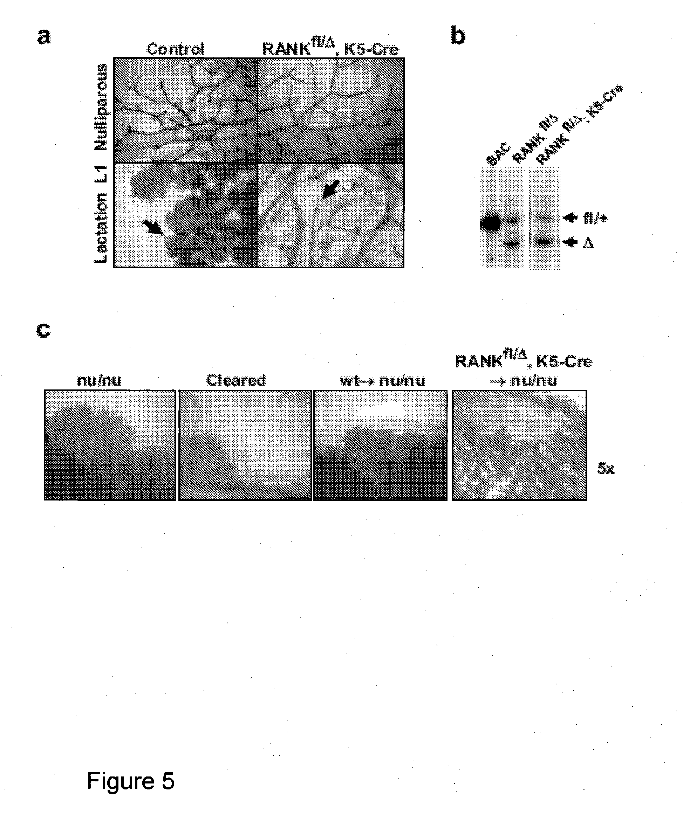

Figure 5. RANK^{fl/Δ} females crossed to the K5-Cre mice show defective lobulo-alveolar development in pregnancy.
   **a,** Whole-mount analyses of mammary tissue of nulliparous and lactating (L1) RANK^{fl/Δ} females crossed with K5-Cre compared to mammary glands of control littermates. Alveoli in gestating wild-type females (arrow) from lobulo-alveolar structures, whereas this development is arrested at a rudimentary alveolar bud (arrow) in K5-Cre RANK^{fl/Δ} females**. b,** Southern blot of purified mammary epithelial cells derived from RANK^{flox/Δ} and RANK^{flox/Δ}; K5-Cre+ females. The wild type or floxed RANK allele (fl/+; 9.6 kb) and the deleted RANK allele (Δ;3.9 kb) are indicated after digestion of genomic DNA with PvuII and SphI. c, Whole-mount analyses of mammary glands from control BALBc nude (*nu*/*nu*) females showing normal lobulo-alveolar structures at day 1 of lactation (L1), normal lobulo-alveolar structures at L1 in "cleared" *nu*/*nu* mice transplanted with wild type mammary gland tissue, and defective lobuloalveolar development at LI in "cleared" *nu*/*nu* mice transplanted with RANK^{fl/Δ}; K5-Cre mammary gland tissue. Fat pads of *nu*/*nu* mice after surgical clearing are also shown. All magnifications X 5.

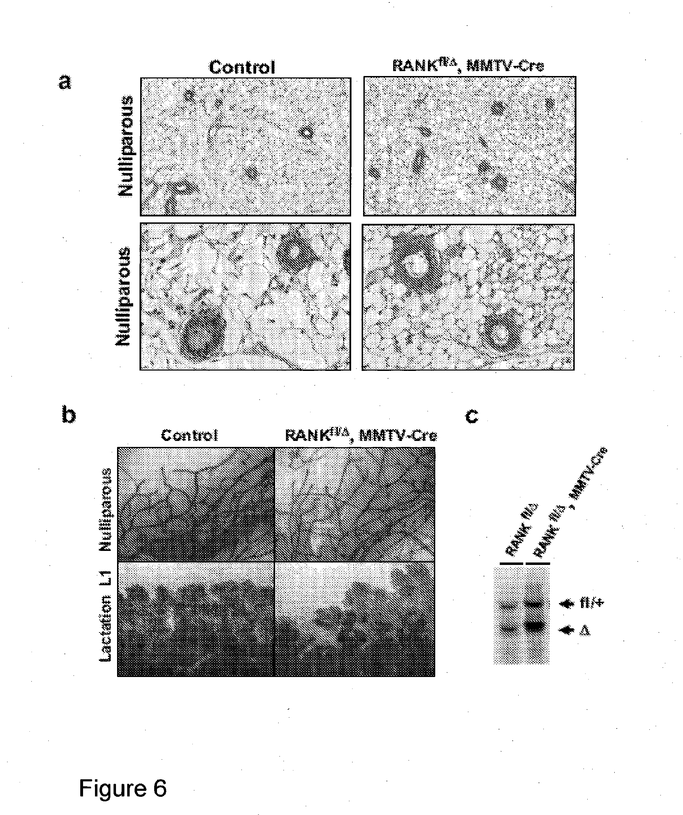

Figure 6. Normal formation of a lactating mammary gland in pregnant MMTV-Cre, RANK^{fl/Δ} females**.**
   **a,** H&E analyses of mammary tissue of nulliparous littermate control and RANK^{fl/Δ}; MMTV-Cre females showing normal alveolar/ductal epithelial structures. Magnifications X 10 (top) and X 40 (bottom panels)**. b,** Whole-mount analyses of mammary tissue of nulliparous and lactating (L1) RANK^{fl/Δ} females crossed with MMTV-Cre compared to mammary glands of control littermates. MMTV-Cre mediated deletion of RANK did not affect formation of a lactating mammary gland**. c,** Southern blot of purified mammary epithelial cells derived from RANK^{flox/Δ} and RANK^{flox/Δ}; MMTV-Cre females. The wild type or floxed RANK allele (fl/+; 9.6 kb) and the deleted RANK allele (Δ;3.9 kb) are indicated after digestion of genomic DNA with PvuII and SphI. RANK^{flox/Δ}; MMTV-Cre animals are denoted RANK^{Δmam} hereafter.

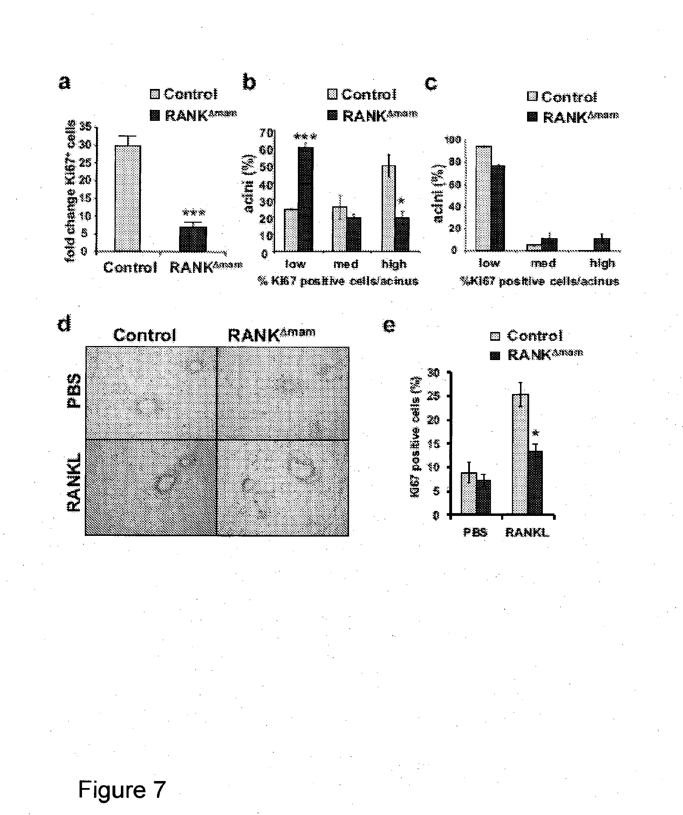

Figure 7. MPA induces RANKL expression and epithelial proliferation in mammary glands.
   **a-c,** Quantification of epithelial proliferation in mammary glands of control littermates and RANK^{Δmam} females 3 days after sham treatment and MPA implantation as shown in Fig. 1d. **a,** Data are shown as relative changes in total Ki67⁺ epithelial cells compared to sham-operated females of the respective genotype. At least 1000 mammary gland epithelial cells were counted per mouse. n = 3 mice per genotype. ** P < 0.005; *** P < 0.001 (Student's t-test)**. b,** Quantification of in situ Ki67 immunostained cells per acinus from mammary glands of control littermate and RANK^{Δmam} females on day 3 after MPA s.c. implantation. Whereas in control females ~ 80% of acini showed signs of medium to high proliferation, more than 60% of acini in RANK^{Δmam} females exhibited very low proliferation rates. **c,** To control for estrus-dependent background proliferation levels, superovulated and sham operated control littermate and RANK^{Δmam} females were analysed. At least 1000 cells were counted per mammary gland. n = 3 per genotype. In **b,** and **c,** data are shown as percentage of acini/ducts with low (<20% of epithelial cells are Ki67⁺), medium (20-80% of epithelial cells are Ki67⁺) and high (>80% of epithelial cells are Ki67⁺) numbers of proliferating cells +/- sem. *** P < 0.001; * P < 0.03 (Student's t-test)**. d,** Epithelial proliferation in mammary glands of control littermates and RANK^{Δmam} females 1 day after i.p. injection of PBS or RANKL (1µg). Proliferation was determined *in situ* Ki67 immunostaining. Magnifications X 40. **e,** Quantification of epithelial proliferation 1 day after i.p. injection of PBS or RANKL (1µg). Mean percentages of Ki67 positive cells +/- sem are shown. * P < 0.03; n=5 (Student's t-test).

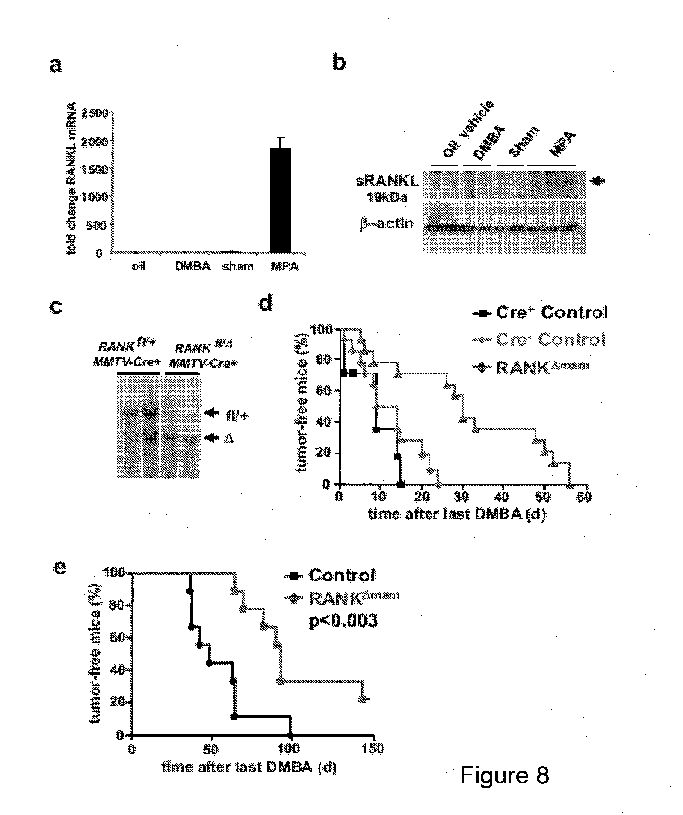

Figure 8. Survival curves of progestin-driven mammary cancers in RANK^{Δmam} mice.
   **a,b,** MPA induces RANKL expression in mammary epithelial cells. Nulliparous wild type females were treated with oral gavage of DMBA or oil vehicle, s.c. implanted with slow-release MPA pellets, or treated with sham surgery. **a,** RANKL mRNA was determined in purified mammary epithelial cells by qRT-PCR 3 days after implantation/oral gavage. Data are shown as fold change compared to sham treatment +/- sem. n=3 mice per group**. b,** Expression of soluble (s) RANKL protein (19kDa) was assayed on cell lysates from purified mammary epithelial cells by Western blot 3 days after treatment with oil vehicle, DMBA, MPA, or sham surgery. β-actin is shown as a loading control. Of note, only MPA but not DMBA or the oil vehicle alone induced RANKL mRNA and protein expression**. c,** Onset of palpable mammary tumors in RANK^{Δmam} (n=14) and age-matched littermate control females with MMTV-Cre (Cre+ control; n=13) or without MMTV-Cre (Cre- control; n=9) treated with MPA pellets and DMBA as indicated in Fig. 2a. Data are shown as percentage of tumor-free mice after the last DMBA challenge. No significant difference was found between the Cre+ and the Cre- control groups. Median tumor onset for Cre⁺ controls was 9 days, for Cre⁻ controls 11 days, and for RANK^{Δmam} females 30 days after the last DMBA treatment**. d,** Representative Southern blot of MPA/DMBA-induced mammary tumors derived from RANK^{flox/+}; MMTV-Cre+ and RANK^{flox/Δ} ; MMTV-Cre+ (RANK^{Δmam}) females. The wild type or floxed RANK allele (fl/+; 9.6 kb) and the deleted RANK allele (Δ;3.9 kb) are indicated after digestion of genomic DNA with PvuII and SphI. All tumors derived from RANK^{Δmam} females showed almost complete deletion. **e,** Kaplan Mayer analysis for overall survival of RANK^{Δmam} (n=9) and age-matched littermate control females (n=9) after treatment with MPA /DMBA. Median survival was 48 days for control and 93 days after the last DMBA treatment for RANK^{Δmam} females.

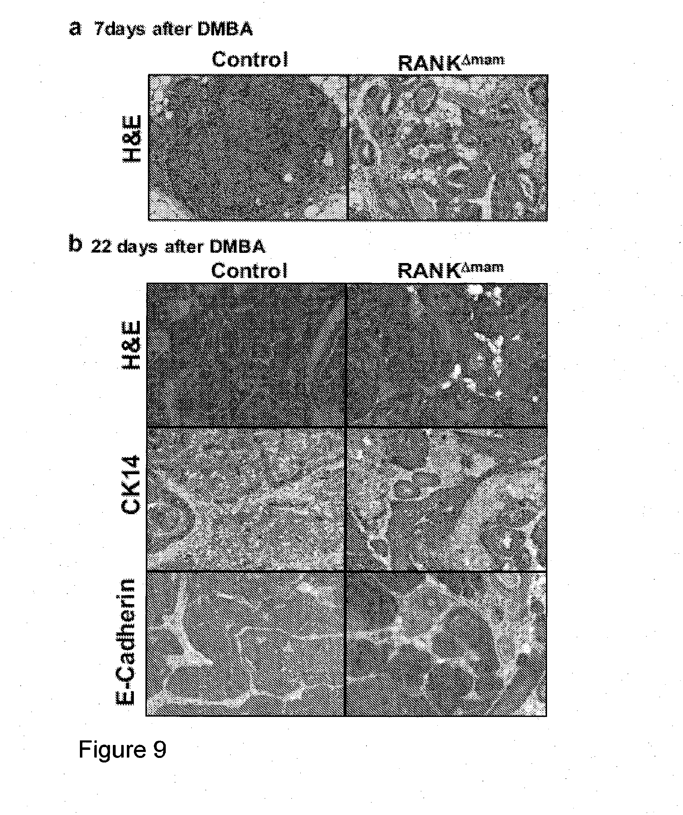

Figure 9. Development of squamous adenocarcinomas in RANK^{Δmam} females.
   **a,b,** Representative histological sections of mammary tumors isolated from control littermate and RANK^{Δmam} females 7 **(a)** and 21 **(b)** days after the last DMBA treatment. H&E and E-cadherin stainings are shown indicating typical features of ductal adenocarcinomas in tumors from control littermates and RANK^{Δmam} females. Cytokeratin14 (K14) expression demonstrates the basal cell origin in both controls and RANK^{Δmam} females. However, RANK^{Δmam} females tend to show characteristics of squamous metaplasia. All magnifications X 20.

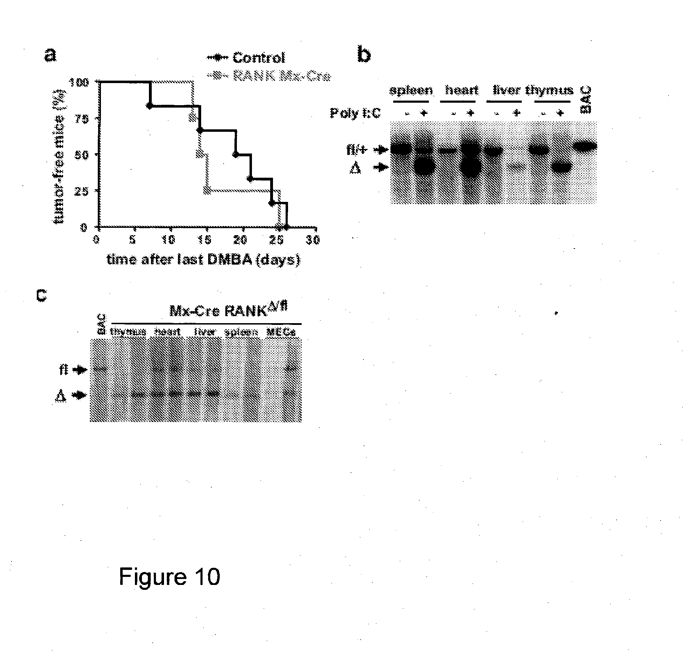

Figure 10. Mammary cancer onset in Mx-Cre RANK^{floxed/Δ} and NeuT RANK^{Δmam} mice.
   **a,** Onset of palpable mammary tumors in Mx-Cre *rank*^{floxed/Δ} (n=4) and age matched Mx-Cre *rank*^{+/Δ} littermate control females (n=6) treated with MPA pellets and DMBA as indicated in Fig. 2a. The Mx-driven Cre recombinase was activated by four poly I:C injections i.p. over the course of 8 days (200µg in 200 ml PBS). Data are shown as percentage of tumor free mice after the last DMBA challenge. No significant differences were found**. b,** Southern blot of the non-deleted RANK^{floxed} allele (fl/+) and after induction of deletion (Δ) in Mx-Cre *rank*^{floxed/Δ} mice**. c**, Southern blot of various organs after induction of deletion (Δ) in Mx-Cre *rank*^{floxed/Δ} mice. While various degrees of deletion (50-100%) can be seen in thymus, heart, liver, and spleen, deletion of the RANK-^{floxed} allele was not induced in purified mammary epithelial cells (MECs).

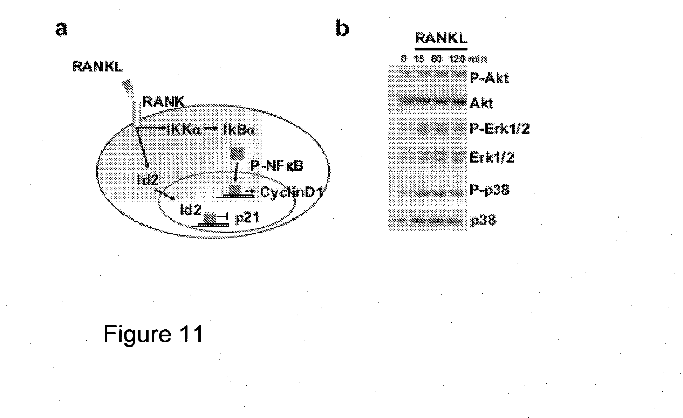

Figure 11. RANKL/RANK downstream signaling in MECs.
   **a,** Schematic outline of genetically confirmed signalling pathways that control RANKL-RANK mediated formation of a lactating mammary gland during pregnancy. **b,** Western blotting for phosphorylated (P) AKT, total AKT, phosphorylated (P) ERK1/2, total ERK1/2, phosphorylated (P) p38-MAPK, and p38-MAPK in isolated primary mouse mammary gland epithelial cells in response to RANKL stimulation (1µg/ml). Data are representative of 4 experiments.

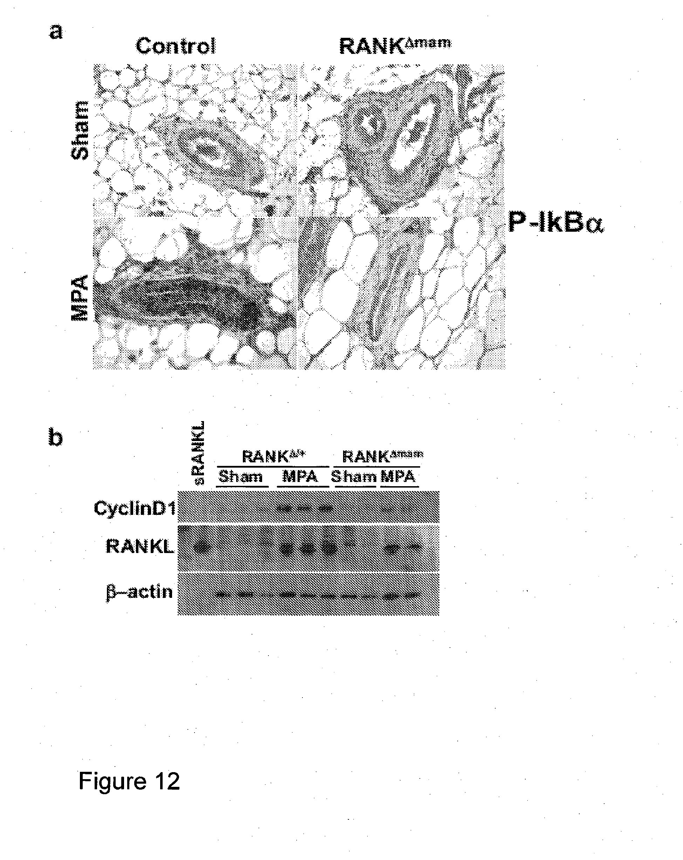

Figure 12. MPA activates the NFκB pathway and triggers CyclinD1 expression via RANKL/RANK.
   **a,** Activation of the NFκB pathway and CyclinD1 expression by MPA. Nulliparous RANK^{Δmam} and littermate control females were s.c. implanted with slow-release MPA pellets or treated with sham surgery. **a,** *In situ* immunostaining to detect phosphorylated (P) IκBα in mammary epithelial cells of RANK^{Δmam} and littermate control females after 3d MPA treatment**. b,** Western blot analysis of CyclinD1 and RANKL in isolated mammary epithelial cells from RANK^{Δmam} and littermate control females after 3d MPA treatment. Recombinant, murine sRANKL protein is shown for molecular size comparison. β-actin is shown as a loading control.

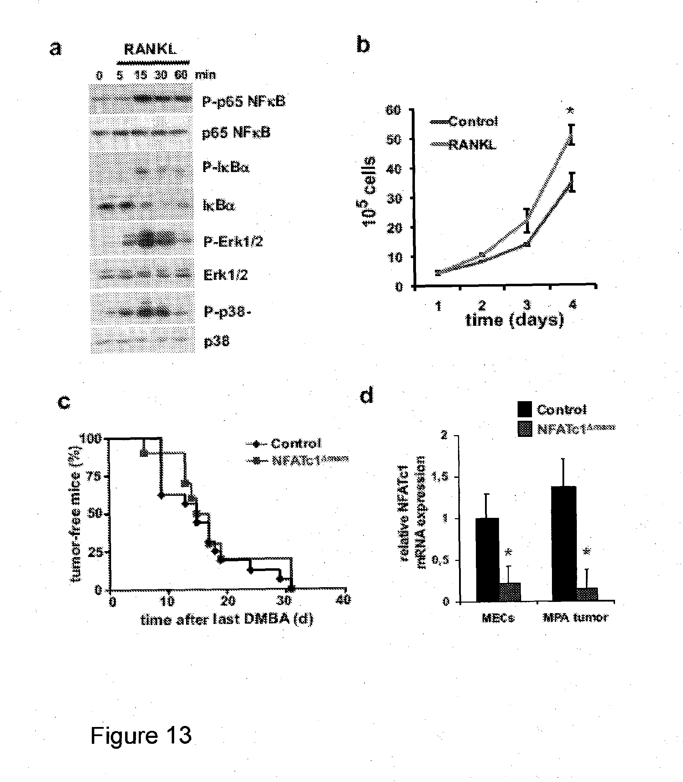

Figure 13. RANKL/RANK downstream signaling pathways.
   **a,** Western blotting for phosphorylated (P) p65 NFκB, total p65 NFκB, phosphorylated (P) IκBα, total IκBα, phosphorylated (P) ERK1/2, total ERK1/2, phosphorylated (P) p38-MAPK, and p38-MAPK in human SKBR3 breast cancer cells in response to RANKL stimulation (1µg/ml). Data are representative of 3 experiments**. b,** Growth curve of SKBR3 breast cancer cells cultured in normal growth medium (control, DMEM supplemented with 10% FCS) or in the presence of RANKL (1µg/ml). Cell numbers were determined by counting live cells (trypan blue-exclusion) over 3 days**. c,** Onset of palpable mammary tumors in NFATc1^{Δmam} (n=10) and age matched littermate control (n=16) females treated with MPA pellets and DMBA. Data are shown as percentage of tumor free mice after the last DMBA challenge. No significant difference was found**. d,** Quantification of NFATc1 mRNA expression in purified mammary epithelial cells (MECs) and MPA-driven mammary tumors from NFATc1^{Δmam} and littermate control females. mRNA was determined by qRT-PCR. Data are shown as fold change compared to control (+/- sem). n=5 Per group. * P < 0.05 (Student's t-test).

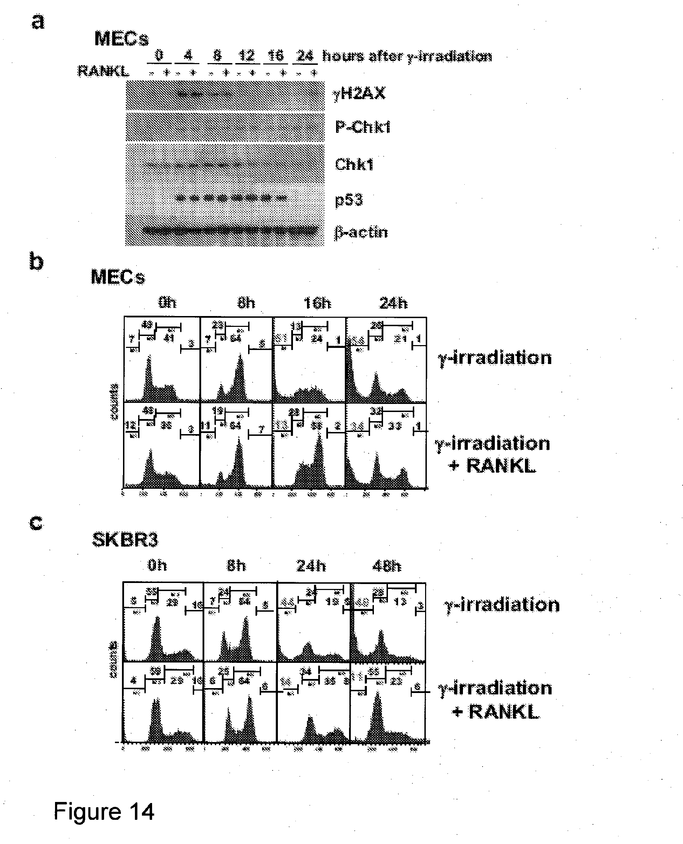

Figure 14. RANKL protects primary murine mammary epithelial cells and human SKBR3 breast cancer cells from apoptosis in response to γ-irradiation.
   **a,** Western Blot analysis for γH2AX, phosphorylated (P) Chk1, total Chk1, p53 and β-actin in isolated primary mouse mammary gland epithelial cells in response to γ-irradiation (2 Gray) in the presence (1µg/ml) or absence of RANKL stimulation**. b,c,** FACS analysis of propidium iodide (PI) stained **b,** mammary epithelial cells and **c,** SKBR3 human breast cancer cells after γ-irradiation (2 Gray) in the absence or presence (1µg/ml) of RANKL. Data are representative of at least 3 experiments. Apoptotic cells with a DNA content < 2n appear in the sub-G1 region. Percent of cells in sub-G1 (M1), G1-phase (M2), S/G2/M-phase (M3) as well as polyploid cells with a DNA content > 4n are given for the indicated time points.

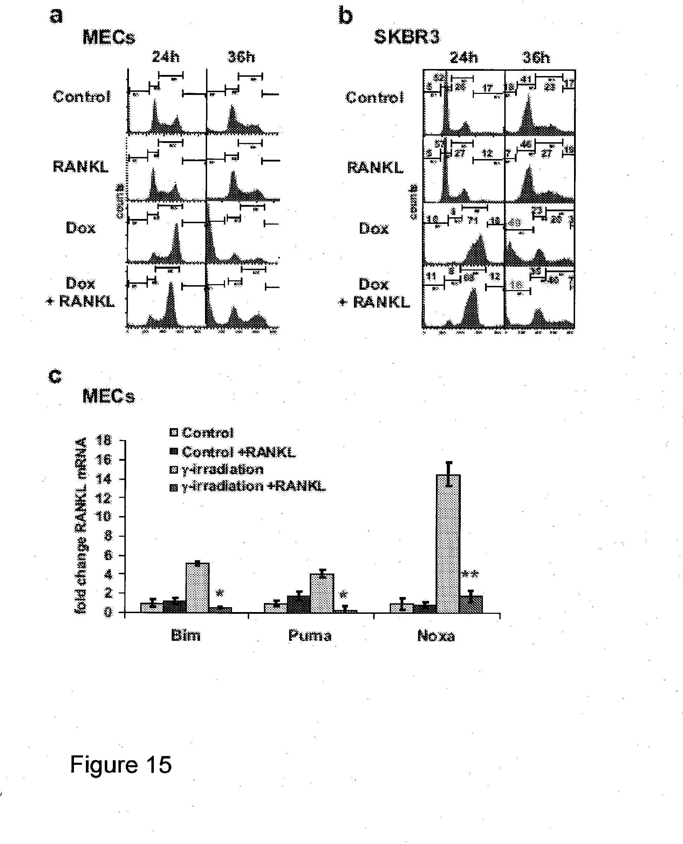

Figure 15. RANKL protects primary murine mammary epithelial cells and human SKBR3 breast cancer cells from apoptosis in response to doxorubicin.
   **a,b,** FACS analysis of **a,** mammary epithelial cells and **b,** SKBR3 human breast cancer cells incubated with the genotoxic agent doxorubicin (Dox, 1µM) in the presence (1µg/ml) or absence of RANKL. Data are representative of at least 3 experiments. Percent of cells in sub-G1 (M1), G1-phase (M2), S/G2/M-phase (M3) as well as polyploid cells with a DNA content >4n are given for 24 and 36 hours after doxorubicin treatment**. c,** mRNA expression of pro-apoptotic genes Bim, Puma, and Noxa 6 hours after γ-irradiation (2 Gray) in the presence (1µg/ml) or absence of RANKL stimulation. Data are shown as fold change compared to control (+/- sem). n=3). * P < 0.05; ** P < 0.005 (Student's t-test).

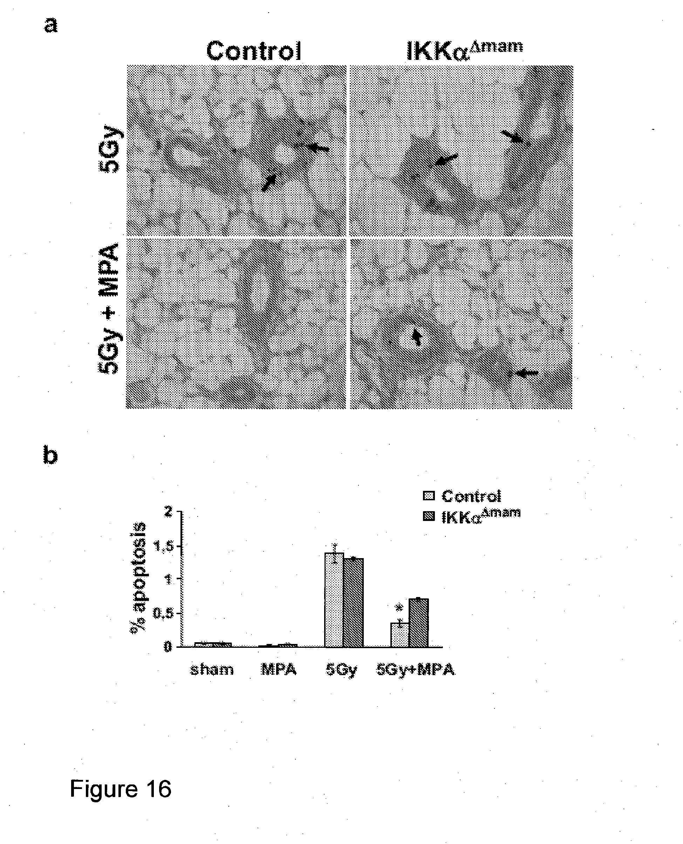

Figure 16. IKKα mediates MPA-induced protection from radiation-induced epithelial apoptosis.
   **a,b,** Reduced induction of mammary epithelial cell apoptosis in response to γ-irradiation in IKKα^{Δmam} females. Littermates control and IKKα^{Δmam} females were either sham operated or implanted with an MPA pellet and γ-irradiated (5 Gray). MPA pellets were implanted 3 days before γ-irradiation. 24 hours after irradiation, apoptosis was detected by immunostaining for active Caspase 3. **a,** Apoptotic nuclei of epithelial cells (arrows) are shown for representative mammary gland sections. Magnifications X 40. **b,** Quantification of mammary epithelial apoptosis. A minimum of 5000 nuclei was counted for each mouse. Results shown are mean values +/- sem. n = 3 mice per group. * P < 0.05 (Student's t-test).

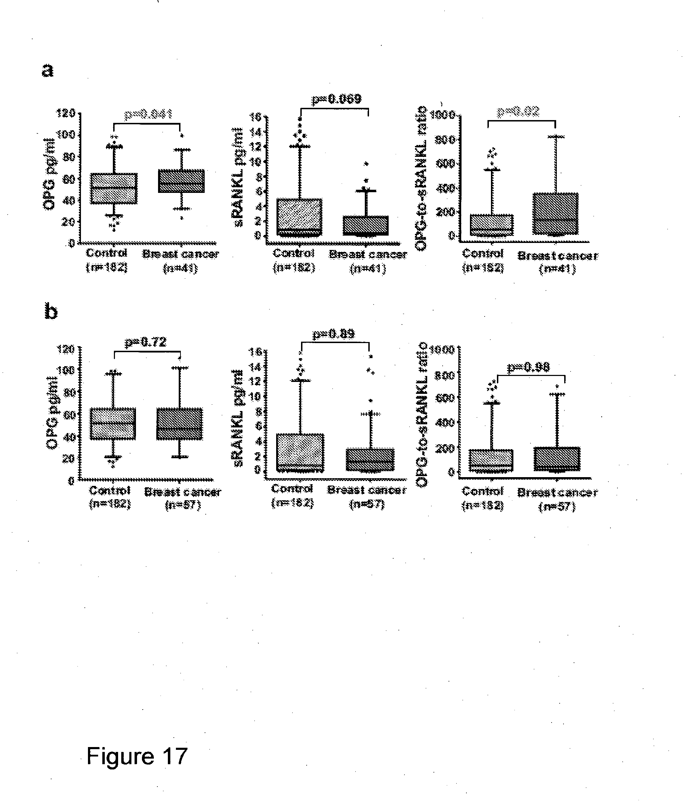

Figure 17. RANKL/OPG ratios are changed in women that develop breast cancer within 5-12 month but not within 12-24 month after serum sampling.
   **a,** Analysis of individual RANKL and OPG levels in prospectively collected serum samples from UKCTOCS (UK Collaborative Trial of Ovarian Cancer Screening) from 182 healthy postmenopausal women who did not develop breast cancer during their follow up and 41 healthy age-matched women who did develop ER positive breast cancer 5-12 months after their serum was collected. Development of breast cancer was diagnosed by biopsies. None of these women was on hormone replacement therapy at the time of sample collection. Box plots of OPG and sRANKL levels as well as OPG-to-sRANKL ratio are shown. Women who developed breast cancer within the first year after the serum was tested have significantly higher OPG levels (p=0.041; Mann Whitney U test) as well as significantly increased OPG-to-sRANKL ratios (p=0.022; Mann Whitney U test) compared to women who did not develop breast cancer**. b**, Analysis of individual RANKL and OPG levels in prospectively collected serum samples from UKCTOCS from 182 healthy postmenopausal women who did not develop breast cancer during their follow up and 57 healthy age-matched women who did develop ER positive breast cancer 12-24 months after their serum was collected. Box plots of OPG and sRANKL levels as well as OPG-to-sRANKL ratios are shown. There were no significant differences (Mann Whitney U test). Box plots indicate median ratio levels and inter-quartile ranges.
Figure 18. Quantification of Western blots.
   Densitometry was performed on at least three independent Western blots per experiment. Data are shown for Western blots in Fig. 1b, Fig. 1c, Fig. 3a, and Fig. 3b. Expression values for the indicated proteins were normalized to β-actin loading controls. For quantification of phosphorylation data were normalized to the respective total protein bands. * P <0.05; ** P < 0.001 (Student's t-test).

### Examples :

Example 1: **Mice.** *Rank*^{floxed} mice have been recently generated¹. Briefly, to generate mice carrying a null allele of *Rank* (*rank*^{Δ} allele), *rank*^{floxed} mice were crossed to β-actin-Cre ubiquitous deleter mice. Mice carrying the *rank*^{floxed} or rank^{Δ} alleles as well as the MMTV-Cre mice were backcrossed seven times onto a BALBc background before generating the MMTV-Cre *rank*^{Δ/floxed} mice. MMTV-NeuT mice were kindly provided by Guido Forni, Milan. MMTV-Cre (stock # 003553) and Mx-Cre mice (stock # 003556) were obtained from the Jackson Laboratory. K5-Cre, *IKKα*^{floxed} and *NFATc1*^{floxed} mice have been previously described²⁻⁴. Mouse genotypes were determined by PCR and Southern blot analysis. In all experiments, only littermate mice from the same breedings were used. All mice were bred and maintained according to institutional guidelines.

**RANK deletion in tumors and Cre effects.** Southern blotting of the tumors that developed in RANK^{Δmam} females showed deletion of RANK, albeit some residual wild type band was observed (Fig. 8c) that may be explained by the presence of other cell types and/or escaper cells. Differences in tumor onset in Cre-negative control females and littermates expressing the MMTV-Cre transgene were not observed indicating that Cre expression per se does not alter tumor incidence in the MPA/DMBA mammary tumor model (Fig. 8d).

### Example 2: MPA/DMBA-induced mammary carcinogenesis.

MPA/DMBA treatment was performed as described^{5,6}. Briefly, six-week old female mice were anesthetized with ketamine-xylazine and surgically implanted with slow-release Medroxyprogesterone Acetat (MPA) pellets (50mg, 90-day release; Innovative Research of America) subcutaneously on the right flank. 200µl DMBA (5mg/ml diluted in cottonseed oil) was administered by oral gavage 6 times throughout the following 8 weeks as outlined in Fig. 2a. Onset of mammary tumors was determined by palpation. Differences in tumor onset in Cre-negative control females and littermates expressing the MMTV-Cre transgene were not observed indicating that Cre expression per se does not alter tumor incidence in the MPA/DMBA mammary tumor model.

### Example 3: Mammary tissue transplants.

For transplantation studies, mammary epithelial tissue was isolated from nulliparous 3-week-old donors and implanted into cleared mammary fat pads (devoid of endogenous epithelium) of 3-week-old host *nu*/*nu* mice as described⁷. Three weeks after surgery, hosts were mated and mammary tissue was isolated for analysis.

Example 4: **Histology, whole-mount, and immunohistochemistry.** For histological analysis, 5µm sections were cut and stained with hematoxylin and eosin (H&E). Whole-mount staining of mammary glands was performed as described⁸. For immunoperoxidase staining paraffin-embedded sections were dehydrated and antigenic epitopes exposed using a 10 mM citrate buffer or microwaving. Sections were incubated with antibodies to cytokeratine 5, cytokeratine 14, E-cadherin, anti-Ki67 (Novocastra) and anti-active Caspase 3 (Cell Signaling) and visualized using peroxidase-conjugated secondary antibodies. Histomorphometric indices (proliferation and apoptosis) were calculated as the number of positive epithelial cells divided by the total number of epithelial cells, with no fewer than 1000 nuclei for Ki67 stainings and no fewer than 5000 cells for active Caspase 3 staining counted per section.

### Example 5: Western blotting.

The human epithelial breast tumor cell line SKBR3 and primary non-transformed mouse mammary epithelial cells were left untreated or stimulated with recombinant murine RANKL^{ref. 9}. Adenocarcinomas were isolated from control and mutant mice and total protein lysates prepared. Western blotting was carried out using standard protocols. Briefly, blots were blocked with 5% BSA in 1xTBS 0.1% Tween-20 (TBST) for 1 hour and incubated with primary antibody overnight at 4°C (diluted in TBST according to the manufactures protocol). Primary antibodies reactive to mouse RANKL (AF462; R&D), Cyclin D1 (Santa Cruz #Sc-8396), β-actin (Sigma), phosphorylated (P) NFκB (#3033), NF-κB (#4767), phosphorylated (P) IκBα (#2859), IκBα (#4814), phosphorylated (P) IKKα (#2681), IKKα (#2678), IKKβ (#2678), IKKγ (#2685), phosphorylated (P) Akt (#3787), Akt (#9272), phosphorylated (P) Erk1/2 (#9101), Erk1/2 (#9102), and p38-MAPK (#9212), p53 (#2524), phosphorylated (P) Chk1 (#2348), Chk1 (#2345) (all from Cell Signaling), p38-MAPK (AF869; R&D), and γH2Ax (Ser139 #07-164 Millipore) were used. Blots were washed 3 times in TBST for 30 minutes, incubated with HRP-conjugated 2^{nd} antibodies (1:2000, Promega) for 1 hour at room temperature, washed 3 times in TBST for 30 minutes, and visualized using ECL.

### Example 6: qRT-PCR.

Total RNA of tumors was prepared using the RNeasy Mini Kit (Qiagen), according to the manufacturer's instructions. Total RNA (2 µg) was subjected to quantitative (q)RT-PCR analysis. The following primers were used:
β-actin forward primer: 5'- GCTCATAGCTCTTCTCCAGGG -3';
β-actin reverse primer: 5'-CCTGAACCCTAAGGCCAACCG -3'.
RANKL forward primer: 5' - CTGAGGCCCAGCCATTTG -3'
RANKL reverse primer: 5' - GTTGCTTAACGTCATGTTAGAGATCTTG -3'
RANK forward primer: 5' - CTTGGACACCTGGAATGAAG -3'
RANK reverse primer: 5' - CAGCACTCGCAGTCTGAGTT -3'
CyclinD1 forward primer: 5' - CTGTGCGCCCTCCGTATCTTA-3'
CyclinD1 reverse primer: 5' - GGCGGCCAGGTTCCACTTGAG-3'
p21 (Cdkn1a) forward primer: 5' - GTGGCCTTGTCGCTGTCTT -3'
p21 (Cdkn1a) reverse primer: 5' - GCGCTTGGAGTGATAGAAATCTG -3'
tRANKL forward primer: 5' - GCGCCGGGCCAGCCGAGACTAC-3'
RANKL1 forward primer: 5' - GTCCCACACGAGGGTCCGCTGC-3'
RANKL2 forward primer: 5' - TGCGCACTCCGGCGTCCCG-3'
RANKL3 forward primer: 5' - CCGAGACTACGGCGGATCCTAACAG-3'
RANKLcom. reverse primer: 5' - TCAGTCTATGTCCTGAACTTTGAAAGCCCC-3'
Puma forward primer: 5' -CCGCCTGATGCCCTCCGCTGTAT -3'
Puma reverse primer: 5' -CGGGCCCACTCCTCCTCCTCCAC -3'
Noxa forward primer: 5' -ACTTTGTCTCCAATCCTCCG -3'
Noxa reverse primer: 5' - GTGCACCGGACATAACTGTG-3'
Bim forward primer: 5' - GTTGAACTCGTCTCCGATCC-3'
Bim reverse primer 5' - GCCCCTACCTCCCTACAGAC-3'

### Example 7: DNA damage responses.

For measurement of cell cycle arrest and apoptosis primary mouse mammary epithelial cells and SKBR3 human breast cancer cells were seeded at a cell-density of 100000 cells/well in a 6-well plate and allowed to grow for 24 hours. Cells where then treated with doxorubicin (1µM) or γ-irradiation (2 Gray) in the absence or presence of recombinant RANKL (1µg/ml). Cell cycle arrest and numbers of dead cells were determined using propidium iodine staining. To determine *in vivo* mammary gland epithelial cell death, control and RANK^{Δmam} littermate females were γ-irradiated with a total dose of 5 Gray (Gy). Six hours later, mammary glands were isolated and immunostained for active Caspase 3 (Cell Signaling) indicative of apoptosis.

### Example 8: Prospective human population studies.

For recruitment details in the prospective population-based UKCTOCS Study see ref. ¹⁰. The subjects were participants in the United Kingdom Collaborative Trial of Ovarian Cancer Screening (UKCTOCS), the largest multi-centre randomised controlled trial for ovarian cancer. The trial was set up at 13 NHS trusts in England, Wales and Northern Ireland and is coordinated by the Gynecological Cancer Research Centre at UCL. Women aged 50-74 were randomly invited from age/sex registers of the 27 participating Primary Care Trusts. Women who accepted the invitation were provided with written and verbal information about the trial. Between 2001 and 2005, a total of 202 638 post-menopausal women aged 50-74 years were randomly assigned to screening for ovarian cancer or no screening. All women provided a blood sample at recruitment and in addition 50 640 women gave annual serial blood samples. This trial is registered as ISRCTN22488978 and with ClinicalTrials.gov, number NCT00058032. Written consent was obtained which included access to their medical records and use of their data/samples in future studies. Each woman provided a sample at recruitment and filled in a baseline questionnaire regarding medical and family history. A follow-up questionnaire was send to the participating women 3.5 years after recruitment requesting whether they developed any cancer after joining the trial. Women who self-reported breast cancer on the follow-up questionnaire or were flagged by the Office of National Statistics (ONS) to have developed breast cancer were further followed-up through their treating physician. Women diagnosed with ER-positive invasive breast cancer, not having HRT treatment at recruitment and having a serum sample given at least 5-24 months prior to diagnosis following randomization into the trial were chosen for analysis (98 cases). Breast cancer cases were age matched and matched with women who had no history of breast cancer (182 controls) and had their serum sample collected on the same day and clinic. The blood samples collected at the centers were spun at 2,500 rpm for 5 minutes and shipped overnight in Grienger gel tubes and processed the next day in the central UKCTOCS laboratory. Serum was aliquoted in 500 microliter straws which were then heat sealed, bar coded, and stored in special containers in liquid nitrogen tanks. The samples were thawed only before use. Ethical approval was provided by the Joint UCL/UCLH Committees on the Ethics of Human Research (REC reference number: 06/Q0505/102).

### Example 9: Breast cancer patients.

The UKCTOCS (UK Collaborative Trial of Ovarian Cancer Screening) cohort¹⁰ used comprises prospectively collected serum samples from from 182 healthy postmenopausal women who did not develop breast cancer during their follow up and 98 healthy age-matched women who did develop estrogen receptor (ER) positive breast cancer 5-24 months after their serum was collected. Of these 98 women, 41 developed breast cancer within the first year and 57 women developed breast cancer 12-24 months after serum sample collection. None of these women was on hormone replacement therapy at the time of sample collection.

### Example 10: Progesterone assay

- 1^{st} incubation: 30 µL sample - in the presence of a biotinylated monoclonal progesterone-specific antibody and a progesterone derivative labeled with ruthenium complex - are incubated with Danazol to release progesterone. Progesterone from the sample competes with the labeled progesterone derivative for the antibody binding site.
- 2^{nd} incubation: After addition of streptavidin-coated microparticles, the complex becomes bound to the solid phase via interaction of biotin and streptavidin. The amount of the labeled progesterone derivative bound to the solid phase is inversely proportional to the progesterone content of the sample.
- The reaction mixture is aspirated into the measuring cell where the microparticles are magnetically captured onto the surface of the electrode. Unbound substances are then removed with ProCell. Application of a voltage to the electrode then induces chemiluminescent emission which is measured by a photomultiplier.
- Results are determined via a calibration curve which is instrument-specifically generated by 2-point calibration and a master curve provided via the reagent barcode.

### Reagents - working solutions

M Streptavidin-coated microparticles (transparent cap), 1 bottle, 6.5mL: Streptavidin-coated microparticles, 0.72mg/mL; preservative.

R1 Anti-progesterone-Ab~biotin (gray cap), 1 bottle, 10 mL: Biotinylated monoclonal anti-progesterone antibody (mouse) 0.15mg/L, phosphate buffer 25mmol/L, pH 7.0; preservative.

R2 Progesterone-peptide∼Ru (bpy)²⁺ (black cap), 1 bottle, 8 mL: Progesterone (of vegetable origin) coupled to a synthetic peptide labeled with ruthenium compley, 10 ng/mL; phosphate buffer 25 mmol/L, pH 7.0; preservative.

Example 11: **Statistics.** All values herein are given as means sem. Comparisons between groups were made by Student's t-test. For the Kaplan-Meier analysis of tumor onset a log rank test was performed. P 0.05 was accepted as statistically significant. In addition to the Log RANK test a post-hoc power analysis was performed (PS Power and Sample Size Calculations, in the web at biostat.mc.vanderbilt.edu/PowerSampleSize) to calculate the probability of correctly rejecting the null hypothesis of equal tumor onset times given the number of experimental animals. For the study involving the RANK^{Δmam} animals the null hypothesis can be reject with a probability (power) of 0.933 and for the IKKα^{Δmam} animals with a probability of 0.766. The Type I error probability associated with this test of this null hypothesis is 0.05. Human data were analysed using a paired t-Test, the Mann Whitney U test, or a Spearman rank test as indicated.

### Example 12: RANKL/RANK impaired mice:

To examine the potential role of RANKL/RANK in tumorigenesis RANK was deleted in mammary epithelial cells using K5-Cre and MMTV-Cre mediated excision of an inducible RANK allele (K5-Cre *rank*^{flox/Δ} mice and MMTV-Cre *rank*^{flox/Δ} mice). Both mouse lines appear healthy and exhibit normal bone structures and lymph node formation. As expected K5-Cre *rank*^{flox/Δ} mice exhibited apparently normal mammary gland development in puberty; however, these mice did not develop milk-secreting lobuloalveolar structures during pregnancy (Fig. 5a,b). These effects were cell autonomous using transplantation experiments (Fig. 5c). In MMTV-Cre *rank*^{flox/Δ} mice, mammary gland development in nulliparous females and formation of milk-secreting lobuloalveolar structures in pregnancy appeared normal (Fig. 6a-c). To exclude any issue of development effects in K5-Cre *rank*^{flox/Δ} mice that might affect certain cell populations in normal physiology, MMTV-Cre *rank*^{flox/Δ} mice were therefore used for all further experiments. These MMTV-Cre *rank*^{flox/Δ} mutant mice are hereafter termed RANK^{Δmam}.

### Example 13: Mechanism of RANKL activation upon progestin administration:

In a wild type population MPA (a progestin) treatment triggers massive proliferation of mammary epithelial cells. MPA-induced proliferation of mammary epithelial cells was significantly reduced in RANK^{Δmam} females (Fig. 1a; Fig. 7a-c). Accordingly, RANKL i.p. injections into nulliparous females triggered proliferation of mammary gland epithelial cells via RANK (Fig. 7d,e). Recently it has been reported that endogenous progesterone affects the numbers of Lin-CD24+CD49f^{hi} stem cells during pregnancy¹⁵ and the estrous cycle¹⁶. In both studies, the RANKL/RANK system was implicated based on *in vivo* whole body Ab blocking studies and RT-PCR expression, however, it was not known whether this is a direct effect of RANKL-RANK in mammary epithelial cells rather than a secondary effect. Therefore it was assayed whether progestins such as MPA can also expand Lin-CD24+CD49f^{hi} cells. MPA treatment resulted in a two-fold expansion of Lin-CD24+CD49f^{hi} cells. Such expansion did not occur in MPA-treated RANK^{Δmam} females (Fig. 1b, c). These data provide the first genetic proof that the RANKL/RANK system controls expansion of Lin-CD24+CD49f^{hi} cells.

### Example 14: Influence of progestins on cancer development via RANK/RANKL in control and RANK/RANKL deficient mice:

In The Women's Health Initiative (WHI) and the Million Women Study, the use of progestins has been epidemiologically linked to the onset and incidence of breast cancer. Progestin-driven mammary cancer can be modeled in female mice, where implantation of slow release MPA pellets in the presence of the DNA damaging agent dimethylbenz[a]anthracene (DMBA) triggers mammary cancer (Fig. 2a; Fig. 8a,b).

In control females, MPA/DMBA treatment induced a rapid onset of palpable mammary tumors. Intriguingly, in RANK^{Δmam} female mice, a marked delay in the onset of MPA/DMBA-induced mammary cancer was observed (Fig. 2b; Fig. 8c,d). Delayed tumor onset on RANK^{Δmam} females also resulted in markedly enhanced survival (Fig. 8e). Southern blotting of the tumors that developed in RANK^{Δmam} females confirmed efficient deletion of RANK (Fig. 8c). All tumors that developed in control and RANK^{Δmam} females exhibited typical histopathology of E-Cadherin expressing ductal adenocarcinomas of the Cytokeratine (CK) 5 and CK14 positive basal cell subtype (Fig. 2c, Fig. 9a,b). However, ductal adenocarcinomas arising in RANK^{Δmam} females frequently developed extensive areas of squamous metaplasia (Fig. 2c, Fig. 9a,b).

### Example 15: Cancer onset after DNA damage in dependence of RANKL mediated progestin signal:

Since RANK^{Δmam} showed a delayed onset in mammary cancer. Next the incidence of mammary tumors at early stages after DMBA challenge was analysed. MPA was again used to simulate a progesterone background to trigger RANKL/RANK signaling. One week after the last DMBA treatment, all RANK expressing control females already exhibited multiple *in situ* carcinomas and even invasive mammary tumors. By contrast, very few carcinomas were observed *in situ* and never any invasive mammary carcinomas in RANK^{Δmam} animals one week after the last DMBA challenge (Fig. 2d). Three weeks after the last DMBA challenge the incidence of carcinomas *in situ* was similar among control and RANK^{Δmam} females, but invasive carcinomas were still very infrequent in the RANK^{Δmam} females (Fig. 2e). Moreover, proliferation was typically reduced in tumors from RANK^{Δmam} females (Fig. 2d,e). Deletion of RANK in multiple other tissues including the liver, heart, muscle and the haematopoietic compartment, but not in mammary epithelial cells, using Mx-Cre *rank*^{flox/flox} mice did not greatly delay the onset of MPA/DMBA-induced mammary cancer (Fig. 10a-c), suggesting that the effects of RANK/RANKL impairment are dominant in mammary epithelial cells.

### Example 16: RANKL signaling:

RANKL-RANK signaling via IKKα-NFκB-CyclinD1 in mammary epithelial cells is illustrated in Fig. 11a. RANKL stimulation indeed resulted in p65 NFκB and IκBα phosphorylation in primary mouse mammary gland epithelial cells (MECs) (Fig. 3a). In addition, RANKL stimulation of MECs triggered phosphorylation of p38-MAPKs and ERK (Fig. 11b). To directly show whether RANK mediates NFκB-CyclinD1 activation downstream of progestins *in vivo,* mice were challenged with MPA. A three day MPA treatment resulted in nuclear accumulation of phosphorylated IκBα, indicative of an active NFκB pathway, and induction of CyclinD1 protein expression in mammary epithelial cells, both of which were severely reduced in RANK^{Δmam} female (Fig. 12a,b). Moreover, in MPA/DMBA-induced mammary adenocarcinomas isolated from control and RANK^{Δmam} females we found impaired activation of the NFκB pathway (Fig. 3b) and downregulated mRNA expression of Cyclin D1 (Fig. 3c). In these primary tumors also upregulation of p21 mRNA (Fig. 3c) was observed, a gene that is transcriptionally suppressed by the Id2 pathway¹⁷. The Id2 pathway is a second genetically confirmed downstream pathway for RANKL/RANK in mammary epithelial cells¹⁷. To extend these findings to human, human SKBR3 breast tumor cells were assayed. RANKL stimulation induced NFκB, p38-MAPKs and ERK activation and proliferation in SKBR3 cells (Fig. 13a,b). To further test the effects of RANKL stimulation the ability of these cells to grow in an anchorage-independent manner was assessed, which correlates well with tumorigenicity *in vivo*¹⁸. Importantly, similar to EGFR stimulation, it was observed that RANKL induced growth of SKBR3 cells in soft agar (Fig. 3d), i.e. RANK signaling not only triggers proliferation but also acts as a transforming agent to induce anchorage-independent growth.

In osteoclasts, besides the NFκB pathway, the calcineurin-NFATc1 signaling pathway has been found to be essential for RANKL-RANK mediated osteoclastogenesis. NFATc1 can also be regulated by the Id2 pathway during RANKL-mediated osteoclastogenesis. To assess whether these key RANKL-RANK activation pathways are also operational in MPA/DMBA-induced mammary cancer, MMTV-Cre *nfatc1*^{flox/Δ} (NFATc1^{Δmam}) and MMTV-Cre *Ikkα*^{flox/flox} (IKKα^{Δmam}) mice were generated to delete NFATc1 and IKKα in mammary epithelial cells. Both mutant mouse strains appear healthy and exhibit no overt defects in any organs assayed. When challenged with MPA/DMBA, IKKα^{Δmam} mice exhibited a delayed onset of mammary cancer (Fig. 3e). In tumors from IKKα^{Δmam} mice normal expression of IKKβ and IKKγ was found but reduced NFκB activation as determined by increased IκB protein levels and decreased p65 NFκB phosphorylation (Fig. 3b) and downregulated mRNA expression of the NFκB target gene Cyclin D1 (Fig. 3c) suggesting that IKKα is indeed required for NFκB signaling in these tumors. As expected, the Id2 pathway gene p21 was not affected in tumors from IKKα^{Δmam} mice (Fig. 3c). No significant differences in the tumor onset between control and NFATc1^{Δmam} mice were observed (Fig. 13c,d), suggesting that downstream signaling requirements are different in osteoclast progenitors and mammary gland epithelial cells. Thus, deletion of IKKα, but not NFATc1, in mammary gland epithelial cells affects the onset of mammary cancer.

### Example 17: Progestin driven cancer development due to DNA damage:

To analyze the role of RANKL in the cellular response to DNA damage such as cell cycle arrest and apoptosis, mouse primary mammary epithelial cells (MECs) and the RANKL-responsive human breast cancer cell line SKBR3 were treated with DNA damaging agents doxorubicin or γ-irradiation. RANKL treatment did not alter induction of γH2AX and p53 or activation of Chk1, prototypic markers of a functional DNA damage response (Fig. 14a). Moreover, RANKL did not alter the early cell cycle arrest after DNA damage with γ-irradiation (Fig. 14b,c) or doxorubicin (Fig. 15a,b). Surprisingly, RANKL treatment resulted in a marked protection from cell death in response to γ-irradiation (Fig. 14b,c) and doxorubicin-induced DNA damage (Fig. 15a,b). Mechanistically, γ-irradiation-induced upregulation of the pro-apoptotic molecules Bim, Puma, and Noxa did not occur in the presence of RANKL (Fig. 15c). *In vivo* it has been shown that γ-irradiation of female mice results in a 5-fold induction of apoptosis of mammary epithelial cells¹⁹. Therefore this previously established system was used to assess the effects of the progesterone-RANKL/RANK axis on γ-irradiation-induced cell death. MPA (a progestin) induced RANKL/RANK indeed protected from γ-irradiation-induced apoptosis of mammary epithelial cells *in vivo.* Loss of RANK expression on mammary epithelial cells abrogated the protective effects of MPA on γ-irradiation-induced cell death (Fig. 3f,g). Moreover, the IKKα pathway was involved in MPA-induced protection of the mammary epithelium after γ-irradiation (Fig. 16a,b). Thus, in addition to promoting cell cycle progression, MPA can protect from cell death after DNA damage via RANKL/RANK and IKKα signaling.

### Example 18: RANKL as diagnostic marker in breast cancer patients:

The data presented herein shows that progestins can induce RANKL/RANK which then drives mammary cancer. To confirm these studies in human breast cancer patients RANKL and progesterone serum levels in women participating in the prospective UKCTOCS (UK Collaborative Trial of Ovarian Cancer Screening) study were analysed (Example 9). This cohort provided a unique opportunity to study changes in serum levels of soluble RANKL, progesterone, and OPG before manifestation of breast cancer. Amazingly, whereas in the control women there was no correlation between progesterone levels and serum RANKL, there was a statistically significant association in women who developed breast cancer 12-24 months after serum collection (p = 0.047, Spearman rank test) (Fig. 4a). In fact, among women within the high progesterone groups, serum RANKL is significantly higher in the group who is going to develop breast cancer (p=0.01, one tailed Wilcoxon rank sum test) (Fig. 4a). Thus, high serum levels of RANKL and high serum progesterone can be used to predict future onset of breast cancer.

The correlation in women who developed breast cancer after 5-12 months after serum sampling was different. Instead, this group showed significant alterations in serum levels of sRANKL and OPG, which was associated with future likelihood to develop breast cancer (Fig. 4b; Fig. 17a). No such alterations with OPG were found in UKCTOCS participants who never developed breast cancer or who developed breast cancer between 12-24 months after serum sampling (Fig. 4c; Fig. 17b).

These serum alterations in RANKL/OPG appear to be counterintuitive. However, this finding is in line with previous data. For instance, it has been reported that in osteoarthritis patients RANKL mRNA levels in the affected bone are positively correlated with bone destruction but inversely correlated with serum RANKL levels¹¹. Moreover, a similar phenomenon was shown for serum RANKL levels and osteoporosis: low serum RANKL levels are associated with a 10-fold higher risk of non-traumatic fractures in postmenopausal women²⁰. Moreover, increased OPG is associated with increased bone loss in postmenopausal women not on hormone replacement therapy²¹. The mechanisms of these serum changes in breast cancer can reflect compensatory mechanisms against developing microtumors and/or redistribution/sequestration of RANKL/OPG within different body compartments. To avoid a misintegration of data in a diagnosis or prognosis it is preferred to compare sample data with positive (patients and/or who developed cancer) on negative (patients who did not develop cancer) controls.

### Example 19: Discussion:

Based on these results the following molecular scenario how progestins such as MPA drive mammary cancer is apparent: Progesterone and progestines (and possibly deregulation of the endogenous progesterone system such as in pre-menopause) trigger an induction of RANKL, foremost in the mammary gland. RANKL via RANK on mammary epithelial cells drives these cells into the cell cycle and, importantly, protects mouse as well as human mammary gland epithelial cells from apoptosis in response to DNA damage including γ-irradiation. Moreover, RANKL-/RANK control self renewal of mammary cancer stem cells and anchorage-independent growth. Thus, progestin-induced RANKL/RANK provide a growth and survival advantage to damaged mammary epithelium, a pre-requisite to initiate mammary cancer²². These effects are, at least in part, mediated via the IKKα-NFκB signalling pathway.

These data have some intriguing implications. For instance, progestins have been associated with an increased risk of having an abnormal mammogram²³. Since mammograms detect micro-calcifications as well as glandular density and RANKL/RANK have key roles in bone metabolism/mineralization^{12,13}, one could speculate that RANKL/RANK contributes to the calcification of such lesions and/or glandular densities. This is of interest because altered RANKL/OPG ratios in the serum of women after and before (5-12 months) the onset of manifest breast cancer were found, proving that RANKL/OPG serum levels are useful biomarkers for the detection of cancer. The data herein also shows that increased RANKL and progesterone serum levels can predict future breast cancer 12-24 months before the tumors is diagnosed. Millions of women take progesterone-derivatives in contraceptives and for hormonal replacement therapy. Such hormones have been epidemiologically linked to an increased risk to develop breast cancer.

### References :

1. Hanada, R. et al. Central control of fever and female body temperature by RANKL/RANK. Nature 462, 505-9 (2009).
2. Tarutani, M. et al. Tissue-specific knockout of the mouse Pig-a gene reveals important roles for GPI-anchored proteins in skin development. Proc Natl Acad Sci U S A 94, 7400-5 (1997).
3. Gareus, R. et al. Normal epidermal differentiation but impaired skin-barrier formation upon keratinocyte-restricted IKK1 ablation. Nat Cell Biol 9, 461-9 (2007).
4. Aliprantis, A.O. et al. NFATc1 in mice represses osteoprotegerin during osteoclastogenesis and dissociates systemic osteopenia from inflammation in cherubism. J Clin Invest 118, 3775-89 (2008).
5. Aldaz, C.M., Liao, Q.Y., LaBate, M. & Johnston, D.A. Medroxyprogesterone acetate accelerates the development and increases the incidence of mouse mammary tumors induced by dimethylbenzanthracene. Carcinogenesis 17, 2069-72 (1996).
6. Cao, Y., Luo, J.L. & Karin, M. IkappaB kinase alpha kinase activity is required for self-renewal of ErbB2/Her2-transformed mammary tumor-initiating cells. Proc Natl Acad Sci U S A 104, 15852-7 (2007).
7. Robinson, G.W. & Hennighausen, L. Inhibins and activins regulate mammary epithelial cell differentiation through mesenchymal-epithelial interactions. Development 124, 2701-8 (1997).
8. Fata, J.E. et al. The osteoclast differentiation factor osteoprotegerinligand is essential for mammary gland development. Cell 103, 41-50 (2000).
9. Lacey, D.L. et al. Osteoprotegerin ligand is a cytokine that regulates osteoclast differentiation and activation. Cell 93, 165-76 (1998).
10. Menon, U. et al. Sensitivity and specificity of multimodal and ultrasound screening for ovarian cancer, and stage distribution of detected cancers: results of the prevalence screen of the UK Collaborative Trial of Ovarian Cancer Screening (UKCTOCS). Lancet Oncol 10, 327-40 (2009).
11. Findlay, D. et al. Circulating RANKL is inversely related to RANKL mRNA levels in bone in osteoarthritic males. Arthritis Res Ther 10, R2 (2008).
12. Kong, Y.Y. et al. OPGL is a key regulator of osteoclastogenesis, lymphocyte development and lymph-node organogenesis. Nature 397, 315-23 (1999).
13. Dougall, W.C. et al. RANK is essential for osteoclast and lymph node development. Genes Dev 13, 2412-24 (1999).
14. Jones, D.H. et al. Regulation of cancer cell migration and bone metastasis by RANKL. Nature 440, 692-6 (2006).
15. Asselin-Labat, M.L. et al. Control of mammary stem cell function by steroid hormone signalling. Nature.
16. Joshi, P.A. et al. Progesterone induces adult mammary stem cell expansion. Nature.
17. Kim, N.S. et al. Receptor activator of NF-kappaB ligand regulates the proliferation of mammary epithelial cells via Id2. Mol Cell Biol 26, 1002-13 (2006).
18. Freedman, V.H. & Shin, S.I. Cellular tumorigenicity in nude mice: correlation with cell growth in semi-solid medium. Cell 3, 355-9 (1974).
19. Ewan, K.B. et al. Transforming growth factor-beta1 mediates cellular response to DNA damage in situ. Cancer Res 62, 5627-31 (2002).
20. Schett, G. et al. Soluble RANKL and risk of nontraumatic fracture. JAMA 291, 1108-13 (2004).
21. Jorgensen, L. et al. Bone loss in relation to serum levels of osteoprotegerin and nuclear factor-kappaB ligand: the Tromso Study. Osteoporos Int 21, 931-8.
22. Hanahan, D. & Weinberg, R.A. The hallmarks of cancer. Cell 100, 57-70 (2000).
23. McTiernan, A. et al. Estrogen-plus-progestin use and mammographic density in postmenopausal women: Women's Health Initiative randomized trial. J Natl Cancer Inst 97, 1366-76 (2005).
24. Terpos et al., Blood 102 (3) (2003): 1064-1069.
25. Hashimoto et al., Cancer & Chemotherapy 7 (31) (2004): 1027-1033; database acc. no. NLM15272580.
26. WO 2005/060627 A2
27. WO 00/43553 A1
28. Beleut et al., PNAS 107 (7) (2010): 2989-2994.
29. Leibbrandt et al., European Journal of Clinical Investigation 39 (10) (2009): 842-850.
30. Reid et al., Molecular Cancer 8 (49) (2009): 1-10.
31. Bo-Ying et al., Annals of Surgical Oncology 17 (6) (2010): 1675-1681.
32. Park et al., J Korean Medical Science 18(4) (2003): 541-546.
33. Chen et al., Cancer 107(2) (2006): 289-298 .

## Claims

1. Method of detecting cancer or of predicting a patient developing cancer, wherein said cancer is breast cancer, comprising determining RANKL activity and the amount of the RANKL ligand osteoprotegerin (OPG) in a sample of said patient.

2. Method according to claim 1, **characterized in that** the RANKL activity is determined by determining the amount of RANKL and/or RANK in the sample.

3. Method according to claim 1, **characterized in that** the RANKL activity is determined by determining signalling of any one of RANK, IKK-alpha, IkB-alpha, P-NF-kappa-B, CyclinDl.

4. Method of any one of claims 1 to 3, **characterized in that** the sample comprises breast cells of the patient.

5. Method according to claim 4, **characterized in that** the cells comprise hormone receptor selected from progesterone receptor and an estrogen receptor.

6. Method according to claim 5, **characterized in that** the estrogen receptor is selected from ESR1, ESR2 or a heterodimer receptor.

7. Method of claim 1 or 2, **characterized in that** RANKL is determined in a serum sample of said patient.

8. Method of any one of claims 1 to 7, **characterized in that** the patient is treated by a hormone or with a hormone contraceptive.

9. Method according to claim 8, **characterized in that** the patient receives hormone replacement therapy, progesterone or a progestin.

10. Method of any one of claims 1 to 6, further comprising determining the serum concentration of a hormone of the menstrual cycle or a progestin in a sample of the patient.

11. The method of claim 10, **characterized in that** the risk of developing breast cancer is estimated by determining at least both the RANKL activity, and progesterone and/or progestin levels, and further the amount of OPG.

12. The method of claim 10, wherein new-onset breast cancer is diagnosed or predicted by determining the ratio of OPG to RANKL.

13. A kit comprising a detection agent for RANKL, a detection agent for OPG, and a detection agent for progesterone or a progestin.

## Patentansprüche

1. Verfahren zum Detektieren von Krebs oder zum Vorhersagen, dass sich bei einem Patienten Krebs entwickeln wird, wobei es sich bei dem Krebs um Brustkrebs handelt, umfassend das Bestimmen einer RANKL-Aktivität und die Menge des RANKL-Liganden Osteoprotegerin (OPG) in einer Probe des Patienten.

2. Verfahren nach Anspruch 1, **dadurch gekennzeichnet, dass** die RANKL-Aktivität durch Bestimmen der Menge von RANKL und/oder RANK in der Probe bestimmt wird.

3. Verfahren nach Anspruch 1, **dadurch gekennzeichnet, dass** die RANKL-Aktivität durch Bestimmen der Signalisierung von einem aus RANK, IKK-alpha, IkB-alpha, P-NF-kappa-B, CyclinD1 bestimmt wird.

4. Verfahren nach einem der Ansprüche 1 bis 3, **dadurch gekennzeichnet, dass** die Probe Brustzellen des Patienten umfasst.

5. Verfahren nach Anspruch 4, **dadurch gekennzeichnet, dass** die Zellen einen Hormonrezeptor umfassen, der aus dem Progesteronrezeptor und einem Östrogenrezeptor ausgewählt ist.

6. Verfahren nach Anspruch 5, **dadurch gekennzeichnet, dass** der Östrogenrezeptor aus ESR1, ESR2 oder einem Heterodimerrezeptor ausgewählt ist.

7. Verfahren nach Anspruch 1 oder 2, **dadurch gekennzeichnet, dass** RANKL in einer Serumprobe des Patienten bestimmt wird.

8. Verfahren nach einem der Ansprüche 1 bis 7, **dadurch gekennzeichnet, dass** der Patient mit einem Hormon oder mit einem Hormonkontrazeptivum behandelt wird.

9. Verfahren nach Anspruch 8, **dadurch gekennzeichnet, dass** der Patient eine Hormonersatztherapie, Progesteron oder ein Progestin erhält.

10. Verfahren nach einem der Ansprüche 1 bis 6, welches ferner das Bestimmen der Serumkonzentration eines Hormons des Menstruationszyklus oder eines Progestins in einer Probe des Patienten umfasst.

11. Verfahren nach Anspruch 10, **dadurch gekennzeichnet, dass** das Risiko der Entwicklung von Brustkrebs abgeschätzt wird, indem mindestens sowohl die RANKL-Aktivität als auch die Progesteron- und/oder Progestinmengen und ferner die OPG-Menge bestimmt werden.

12. Verfahren nach Anspruch 10, wobei neu aufgetretener Brustkrebs diagnostiziert oder vorhergesagt wird, indem das Verhältnis von OPG zu RANKL bestimmt wird.

13. Kit, umfassend ein Detektionsmittel für RANKL, ein Detektionsmittel für OPG und ein Detektionsmittel für Progesteron oder ein Progestin.

## Revendications

1. Procédé de détection du cancer ou de prédiction du développement du cancer chez un patient, où ledit cancer est le cancer du sein, comprenant la détermination de l'activité de RANKL et de la quantité du ligand de RANKL, l'ostéoprotégérine (OPG), dans un échantillon dudit patient.

2. Procédé selon la revendication 1, **caractérisé en ce que** l'activité de RANKL est déterminée en déterminant la quantité de RANKL et/ou de RANK dans l'échantillon.

3. Procédé selon la revendication 1, **caractérisé en ce que** l'activité de RANKL est déterminée en déterminant la signalisation de l'un quelconque de RANK, IKK-alpha, IkB-alpha, P-NF-kappa-B, et la cycline D1.

4. Procédé selon l'une quelconque des revendications 1 à 3, **caractérisé en ce que** l'échantillon comprend des cellules mammaires du patient.

5. Procédé selon la revendication 4, **caractérisé en ce que** les cellules comprennent un récepteur hormonal sélectionné parmi le récepteur de la progestérone et un récepteur des oestrogènes.

6. Procédé selon la revendication 5, **caractérisé en ce que** le récepteur des oestrogènes est sélectionné parmi ESR1, ESR2 ou un récepteur hétérodimère.

7. Procédé selon la revendication 1 ou la revendication 2, **caractérisé en ce que** RANKL est déterminé dans un échantillon de sérum dudit patient.

8. Procédé selon l'une quelconque des revendications 1 à 7, **caractérisé en ce que** le patient est traité par une hormone ou par un contraceptif hormonal.

9. Procédé selon la revendication 8, **caractérisé en ce que** le patient reçoit une thérapie de substitution hormonale, de la progestérone ou une progestine.

10. Procédé selon l'une quelconque des revendications 1 à 6, comprenant en outre la détermination de la concentration sérique d'une hormone du cycle menstruel ou d'une progestine dans un échantillon du patient.

11. Procédé selon la revendication 10, **caractérisé en ce que** le risque de développer un cancer du sein est estimé en déterminant au moins à la fois l'activité de RANKL et les taux de progestérone et/ou progestine, et en outre la quantité d'OPG.

12. Procédé selon la revendication 10, dans lequel un cancer du sein d'apparition récente est diagnostiqué ou prédit en déterminant le rapport OPG/RANKL.

13. Kit comprenant un agent de détection de RANKL, un agent de détection de l'OPG, et un agent de détection de la progestérone ou d'une progestine.


### SEQUENCE LISTING

<110> IMBA - Institut für Molekulare Biotechnologie GmbH
<120> Breast Cancer Diagnostics
<130> R 59662
<150> EP 10178345.4
   <151> 2010-09-22
<160> 21
<170> PatentIn version 3.5
<210> 1
   <211> 21
   <212> DNA
   <213> Artificial Sequence
<220>
   <223> Primer
<400> 1
   gctcatagct cttctccagg g 21
<210> 2
   <211> 21
   <212> DNA
   <213> Artificial Sequence
<220>
   <223> Primer
<400> 2
   cctgaaccct aaggccaacc g 21
<210> 3
   <211> 18
   <212> DNA
   <213> Artificial Sequence
<220>
   <223> Primer
<400> 3
   ctgaggccca gccatttg 18
<210> 4
   <211> 28
   <212> DNA
   <213> Artificial Sequence
<220>
   <223> Primer
<400> 4
   gttgcttaac gtcatgttag agatcttg 28
<210> 5
   <211> 20
   <212> DNA
   <213> Artificial Sequence
<220>
   <223> Primer
<400> 5
   cttggacacc tggaatgaag 20
<210> 6
   <211> 20
   <212> DNA
   <213> Artificial Sequence
<220>
   <223> Primer
<400> 6
   cagcactcgc agtctgagtt 20
<210> 7
   <211> 21
   <212> DNA
   <213> Artificial Sequence
<220>
   <223> Primer
<400> 7
   ctgtgcgccc tccgtatctt a 21
<210> 8
   <211> 21
   <212> DNA
   <213> Artificial Sequence
<220>
   <223> Primer
<400> 8
   ggcggccagg ttccacttga g 21
<210> 9
   <211> 19
   <212> DNA
   <213> Artificial Sequence
<220>
   <223> Primer
<400> 9
   gtggccttgt cgctgtctt 19
<210> 10
   <211> 23
   <212> DNA
   <213> Artificial Sequence
<220>
   <223> Primer
<400> 10
   gcgcttggag tgatagaaat ctg 23
<210> 11
   <211> 22
   <212> DNA
   <213> Artificial Sequence
<220>
   <223> Primer
<400> 11
   gcgccgggcc agccgagact ac 22
<210> 12
   <211> 22
   <212> DNA
   <213> Artificial Sequence
<220>
   <223> Primer
<400> 12
   gtcccacacg agggtccgct gc 22
<210> 13
   <211> 19
   <212> DNA
   <213> Artificial Sequence
<220>
   <223> Primer
<400> 13
   tgcgcactcc ggcgtcccg 19
<210> 14
   <211> 25
   <212> DNA
   <213> Artificial Sequence
<220>
   <223> Primer
<400> 14
   ccgagactac ggcggatcct aacag 25
<210> 15
   <211> 30
   <212> DNA
   <213> Artificial Sequence
<220>
   <223> Primer
<400> 15
   tcagtctatg tcctgaactt tgaaagcccc 30
<210> 16
   <211> 23
   <212> DNA
   <213> Artificial Sequence
<220>
   <223> Primer
<400> 16
   ccgcctgatg ccctccgctg tat 23
<210> 17
   <211> 23
   <212> DNA
   <213> Artificial Sequence
<220>
   <223> Primer
<400> 17
   cgggcccact cctcctcctc cac 23
<210> 18
   <211> 20
   <212> DNA
   <213> Artificial Sequence
<220>
   <223> Primer
<400> 18
   actttgtctc caatcctccg 20
<210> 19
   <211> 20
   <212> DNA
   <213> Artificial Sequence
<220>
   <223> Primer
<400> 19
   gtgcaccgga cataactgtg 20
<210> 20
   <211> 20
   <212> DNA
   <213> Artificial Sequence
<220>
   <223> Primer
<400> 20
   gttgaactcg tctccgatcc 20
<210> 21
   <211> 20
   <212> DNA
   <213> Artificial Sequence
<220>
   <223> Primer
<400> 21
   gcccctacct ccctacagac 20